(19) Europäisches Patentamt
European Patent Office
Office européen des brevets

(11) EP 3 043 276 B1

(12) **EUROPEAN PATENT SPECIFICATION**

(45) Date of publication and mention of the grant of the patent:
**11.03.2020 Bulletin 2020/11**

(51) Int Cl.:
*G16H 40/63* $^{(2018.01)}$ *G16H 50/50* $^{(2018.01)}$

(21) Application number: **15202009.5**

(22) Date of filing: **22.12.2015**

(54) **PERSONALIZED WHOLE-BODY CIRCULATION IN MEDICAL IMAGING**

PERSONALISIERTER GANZKÖRPERKREISLAUF IN DER MEDIZINISCHEN BILDGEBUNG

CIRCULATION DE CORPS ENTIER PERSONNALISÉ EN IMAGERIE MÉDICALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.01.2015 US 201562100271 P**
**17.12.2015 US 201514973345**

(43) Date of publication of application:
**13.07.2016 Bulletin 2016/28**

(73) Proprietor: **Siemens Healthcare GmbH**
**91052 Erlangen (DE)**

(72) Inventors:
- **COMANICIU, Dorin**
**Princeton Junction, NJ 08550 (US)**
- **ITU, Lucian Mihai**
**500294 Brasov (RO)**
- **MANSI, Tommaso**
**Plainsboro, NJ 08536 (US)**
- **MIHALEF, Viorel**
**North Brunswick, NJ 08902 (US)**
- **NEUMANN, Dominik**
**91052 Erlangen (DE)**
- **PASSERINI, Tiziano**
**Plainsboro, NJ 08536 (US)**
- **SHARMA, Puneet**
**Monmouth Junction, NJ 08852 (US)**

(74) Representative: **Patentanwälte Bals & Vogel**
**Sendlinger Strasse 42A**
**80331 München (DE)**

(56) References cited:
- **Radomir Chabiniok: "Personalized Biomechanical Heart Modeling for Clinical Applications", , 24 January 2011 (2011-01-24), XP055415108, Retrieved from the Internet: URL:https://tel.archives-ouvertes.fr/tel-00839929/document [retrieved on 2017-10-12]**
- **RAMAKRISHNA MUKKAMALA ET AL: "A forward model-based validation of cardiovascular system identification", AMERICAN JOURNAL OF PHYSIOLOGY: HEART AND CIRCULATORY PHYSIOLOGY, vol. 281, no. 6, 1 December 2001 (2001-12-01), pages 2714-2730, XP055415351, US ISSN: 0363-6135**
- **SETHURAMAN SANKARAN ET AL: "A Stochastic Collocation Method for Uncertainty Quantification and Propagation in Cardiovascular Simulations", JOURNAL OF BIOMECHANICAL ENGINEERING., vol. 133, no. 3, 1 March 2011 (2011-03-01) , pages 031001-1, XP055415354, US ISSN: 0148-0731, DOI: 10.1115/1.4003259**
- **DARRELL J SWENSON ET AL: "Cardiac Position Sensitivity Study in the Electrocardiographic Forward Problem Using Stochastic Collocation and Boundary Element Methods", ANNALS OF BIOMEDICAL ENGINEERING, KLUWER ACADEMIC PUBLISHERS-PLENUM PUBLISHERS, NE, vol. 39, no. 12, 10 September 2011 (2011-09-10), pages 2900-2910, XP019971849, ISSN: 1573-9686, DOI: 10.1007/S10439-011-0391-5**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 043 276 B1

## Description

RELATED APPLICATIONS

The present patent document claims the benefit of the filing date under 35 U.S.C. §119(e) of Provisional U.S. Patent Application Serial No. 62/100,271, filed January 6, 2015.

BACKGROUND

The present embodiments relate to the estimation of comprehensive parameters related to the whole-body circulation from medical imaging and clinical data.

The capacity of the heart to pump sufficient blood to match its own demands and the demands of the body depends on both intrinsic and extrinsic factors. The modeling of these factors may lead to better approaches to evaluate and manage cardiac disease, as well as better patient stratification and therapy planning. However, many models of whole-body circulation are overly simplified, process intensive, too general (i.e. not reflecting patient's physiology), and/or inaccurate to be of use in clinical settings for assisting a given patient. Radomir Chabiniok: "Personalized Biomechanical Heart Modeling for Clinical Applications", 24 January 2011 (2011-01-24) discloses a personalized whole-body circulation calculation system considering spatial data of a patient, cardiac electrophysiological data of the patient and pressure data of the patient to compute circulation metrics.

BRIEF SUMMARY

The scope of the invention is determined by the appending independent claims. Advantageous embodiments are determined by the appending dependent claims.

BRIEF DESCRIPTION OF THE DRAWINGS

The components and the figures are not necessarily to scale, emphasis instead being placed upon illustrating the principles of the invention. Moreover, in the figures, like reference numerals designate corresponding parts throughout the different views.

Figure 1 is a flow chart diagram of one embodiment of a method for personalized whole-body circulation calculation;
Figure 2 illustrates an example of exchange between cardiovascular and regulatory systems;
Figure 3 illustrates the components of the cardiovascular and regulatory systems of Figure 2;
Figure 4 illustrates one embodiment of a lumped parameter, closed-loop model of the cardiovascular system;
Figure 5 illustrates one embodiment of a combined lumped and three-dimensional, closed-loop model of the cardiovascular system;
Figure 6 illustrates one embodiment of an extended or greater scale lumped systemic and pulmonary model;
Figure 7 is a flow chart diagram of one embodiment of a method for personalization;
Figure 8 shows graphs comparing model-based computation against measured results for a volume-pressure loop;
Figure 9 is a flow chart diagram of one embodiment of a method for personalized, whole-body circulation calculation;
Figure 10 is a flow chart diagram of a further embodiment of the method of Figure 9;
Figure 11 is a flow chart diagram of another further embodiment of the method of Figure 9; and
Figure 12 is a block diagram of one embodiment of a system for personalized whole-body circulation calculation.

DETAILED DESCRIPTION OF THE DRAWINGS AND PRESENTLY PREFERRED EMBODIMENTS

Personalized computation of the whole-body circulation is performed using medical images and signals for a patient. A comprehensive patient-specific multiscale computational model of the cardiovascular system is composed of a full-scale or reduced-order cardiac electro-mechanics model coupled to a whole body circulation model. The multi-scale computational model is used to estimate parameters and calculate dynamics of the heart and the entire cardiovascular system. The parameters to be personalized may be specified a priori or may be identified automatically based on a set of metrics of interest. Once these parameters are known, their personalization is performed automatically. The computed cardiovascular metrics of interest may be used in patient stratification, disease estimation, and/or therapy planning. The resulting computational model is used to test different therapy configurations by computing acute predictors, which are used to determine if the patient will respond to treatment in a planning phase or guide the clinician towards the therapy target in intervention (e.g. placement of the left ventricle (LV) lead for cardiac resynchronization therapy (CRT)).

In one embodiment, cardiovascular images, signals and data, including at least one medical image of a patient, acquisition of ECG, and systolic and diastolic cuff pressures, are acquired. To build the geometry of the heart or at least of one chamber (e.g., left ventricle) in at least two time phases of the cardiac cycle (e.g., peak systole and peak diastole), the images are segmented. Hemodynamic parameters, including time-varying parameterized flow rate functions and pressure variation functions for one or both heart chambers, and cardiovascular systemic and pulmonary impedances are personalized. The multiscale whole-body circulation model dynamics are computed with the personalized parameters. The computed data is visualized as outcome curves, or as scalar and/or vector fields overlaid or displayed as attributes of the segmented geometries or the imaging data.

A comprehensive closed-loop cardiovascular system (CLCS) model is able to simulate physiological and pathophysiological characteristics, and quantify the cardiac workload from those characteristics. This approach enables a better understanding of the complex relationship between heart disease and the extra workload on the heart due to various pathologies such as hypertrophy, cardiomyopathy (arrhythmogenic right ventricular cardiomyopathy, isolated ventricular non-compaction, mitochondrial myopathy, dilated cardiomyopathy, restrictive cardiomyopathy, peripartum cardiomyopathy, takotsubo cardiomyopathy, loeffler endocarditis, etc.), mitral regurgitation, aortic stenosis, aortic regurgitation, and hypertension.

The multiscale cardiac models coupled to circulation models are personalized. A typical use case is the non-invasive computation of left or right ventricular pressure-volume loops, but other diagnostically or therapeutically useful metrics may be computed. Machine learning based workflows may improve a physiological reduced-order model and/or may be used to derive a data-driven forward model with features extracted from a reduced-order physiological model. A full scale or greater scale than the reduced-order model is used in training the machine-learnt classifier.

Figure 2 shows a method for personalized whole-body circulation calculation. The method is implemented by a medical diagnostic imaging system, a review station, a workstation, a computer, a picture and archiving and communications system (PACS) station, a server, combinations thereof, or other device for image processing medical scan data. For example, the system, computer readable media, and/or processor shown in Figure 12 implement the method, but other systems may be used.

The method is implemented in the order shown or a different order. Additional, different, or fewer acts may be performed. For example, act 22 is not performed where the personalization operates on the image data without segmentation. In another example, acts for storing scanned data and/or transfer of results are provided. In yet another example, acts 34, and/or 36 are not provided.

The acts are performed in real-time, such as during a surgical procedure. Performing during the procedure allows the clinician to diagnose and/or treat based on flow information computed from the scan data to assist an ongoing procedure. In other embodiments, the acts are performed after a procedure (e.g., performing as part of a review), as part of diagnosis, or before a procedure for planning. The method may be repeated to provide comparative information over time.

The acts are performed automatically by a processor. The user causes the patient to be scanned or obtains scan data for the patient from a previous scan. The user may activate the process and input patient-specific information, such as a metric of interest, age, sex, and/or weight. Once personalization and/or metric computation are activated, the method is performed without any user input, such as without user input of locations and/or values. Alternatively, the user assists in a semi-automated process, such as the user indicating possible values of properties. Other user input may be provided, such as for changing modeling parameter values, correcting output, and/or to confirm accuracy.

In act 20, data representing a patient is obtained. One or more of different types of data are obtained. For example, data from a computerized medical record is obtained, such as diagnosis, age, weight, and sex. In one embodiment, scan, function, and pressure data are obtained. The function data is ECG measurements measured with electrodes and an ECG sensor, but other function data may be obtained. The pressure data is pressures measured with a pressure cuff, but internal pressures may instead or additionally be measured with a pressure sensor on a catheter. The scan data is image data or spatial data measured with a medical diagnostic scanner, such as an ultrasound, computed tomography, x-ray, fluoroscopy, angiography, or magnetic resonance scanner. Any scanning sequence or approach may be used. Other types of data may be obtained.

The data is obtained at a same time, such as measuring pressure and function while also scanning. Alternatively, the data is obtained at different times, such as in sequence during a single patient visit or appointment or in sequence across hours or days.

The data is acquired by scanning and/or measuring the patient. In an alternative embodiment, the data is acquired by loading from memory. Data from a previously performed scan of the patient is stored in a memory, such as a picture archiving and communications system (PACS) database. The data is selected from the database. The data may be obtained by transfer, such as over a network or on a portable memory device.

The scan data represents a volume. The scan data is organized or formatted as a frame, set of data, sets of data, or other collection to represent the volume. The scan data represents locations distributed in three dimensions. The volume includes the heart and one or more vessels. Only a portion of the heart may be imaged in other embodiments.

Scan data of the volume over time may be acquired.

In act 22, a processor segments the cardiovascular spatial data for a heart of the patient. Scan data representing the heart within a three-dimensional volume of the patient represented in the cardiovascular spatial data is identified. Using thresholding, edge detection, contrast detection, shape fitting, flow detection, combinations thereof, or other process, locations associated with the part or all of the heart or other cardiovascular part as compared to other anatomy are identified. The type of scanning or detection may result in acquiring data from the cardiovascular system and no other anatomy, such as by contrast detection and/or flow detection. The heart may be represented as tissue of the heart walls, the boundary of the tissue with blood, and/or the exterior of the blood column.

To allow for calculation of change or variation through the cardiac cycle, the locations for cardiac structure (e.g., heart chambers) are segmented in at least two phases of a cardiac cycle. The scan data is acquired for each of the phases (e.g., end diastole and end systole). The scan data for each phase is segmented to provide the anatomy at the different times in the cycle.

The obtained data with or without segmentation is used with a multi-scale, whole-body circulation model. Whole-body represents inclusion of representation of both the heart and vessels. Heart, systemic circulation, and pulmonary circulation are accounted for in the modeling. Since the model is a whole-body model of the cardiovascular system, the model is a closed-loop model. Circulation is for blood in the cardiovascular system. Multi-scale indicates that the model includes different levels of information or representation, such as cellular, organ, and circulatory. The cellular may be fibers or other sub-anatomical representation used in the model, such as for modeling electrophysiology of the heart. The organ may be the heart, vessel, or part of the heart or vessel, such as represented by the segmented data. The circulatory of multi-scale represents a system, such as a collection of organs. The multi-scale model includes two or more levels or orders (i.e., scales) of representation, such as two or more of 3D, 2D, 1 D, and 0D.

The model is for one phase, across phases, or at a resting state. In one embodiment, different patient states (steady state and transient) are modeled. Figure 2 shows the cardiovascular model coupled to one or more (e.g., a series of) models representing the cardiovascular regulatory systems. The bidirectional exchange of information between the systems leads to a continuous adaptation of the cardiovascular activity and operation. In modeling, the flow rate or volume change and pressure data from the cardiovascular model are provided to the regulatory system model and the regulatory system model returns adapted or altered values for use in the cardiovascular model.

In act 24, a processor personalizes parameters of the closed-loop or whole-body cardiovascular model. One or more values for parameters used in the model are set based on the obtained data of act 20 for the patient. For example, the segmented cardiovascular spatial data, the ECG data, and the pressure data are used to determine values for multiple parameters used in the model.

Example parameters include time-varying flow rate for the heart, pressure variation for the heart, cardiovascular systemic impedance, and cardiovascular pulmonary impedance. Values for these parameters at one or multiple locations in the cardiac system are used in the model. Any number of parameters and correspondingly any number of values over time and/or space for each parameter may be used. Other example parameters used in the modeling may include systolic aortic pressure [mmHg], diastolic aortic pressure [mmHg], heart rate [bpm], ejection fraction [%], end-diastolic volume [ml], stroke volume [ml], left ventricular end-systolic pressure [mmHg], left ventricular end-systolic elastance [mmHg/ml], arterial compliance, volume (V), $V_0$,* [ml] (dead volume of the * chamber of the heart), $V_{100}$ [ml](left ventricular volume corresponding to a left ventricular pressure of 100mmHg), proximal arterial resistance [g/(cm$^4$·s)], distal arterial resistance [g/(cm$^4$·s)], total arterial resistance [g/(cm$^4$·s)], stroke work PV [J] (stroke work determined from computed PV loop), normalized stroke work PV [J/ml] (stroke work PV divided by stroke volume), stroke work PQt [J] (stroke work determined from computed ventricular pressure and aortic flow rate), normalized stroke work PQt [J/ml] (stroke work PQt divided by stroke volume), arterial elastance [mmHg/ml] (computed as end systolic pressure divided by stroke volume), and/or arterial ventricular coupling (arterial elastance divided by left ventricular end-systolic elastance). Additional, different, or fewer parameters may be used in the modeling. The parameters are of input variables used to model. Alternatively, one or more of the above listed variables are output metrics calculated from the model as personalized.

The one or more personalized parameters are used in any closed-loop, cardiovascular system model. In one embodiment, the closed-loop cardiovascular system model is a lumped parameter or multiscale model of the cardiovascular system. Figure 3 shows the closed loop cardiovascular system and regulatory system of Figure 2 in more detail. The model may represent these systems.

Various regulatory systems that act on the cardiovascular system are presented in Figure 3. The objectives of these regulatory systems are to maintain certain levels of blood pressure, flow rate to a certain organ, body temperature, filtration rate, or oxygen level in the blood. Specifically, most systems of the body show some degree of autoregulation. The heart and the brain are very sensitive to over- and under-perfusion, so regulation controls the amount of perfusion. Coronary autoregulation ensures that the coronary blood supply matches the oxygen demand of the myocardium, both at rest and at exercise (hyperemia), by adapting the resistance of the coronary microvasculature. Cerebral autoregulation also focuses on maintaining an appropriate blood flow to the subtended cerebral tissue.

Blood pressure regulation at the systemic level is performed by the baroreflex system, which uses input data provided by the baroreceptors situated mainly in the aortic arch and at the carotid sinuses, and by the renin-angiotensin system, which is triggered by pressure and flow receptors in the afferent arterioles of the renal arterial circulation. The renal autoregulation system adapts the resistance of the renal microvasculature in order to maintain the reference glomerular filtration rate. Additional, different, or fewer regulatory systems may be modeled. The regulatory systems are modeled by differential equations. For example in the baroreflex system, different mathematical functions are used, such as:

1. $R_{syst}$ = f (Pa), where $R_{syst}$ is the total systemic resistance and Pa is the mean arterial pressure and 2. $C_v$ = f (Pa) where $C_v$ is the venous compliance. but Other approaches may be used

The whole-body cardiovascular system model contains a heart model (left and right side of the heart, each of them with atrium and ventricle), the systemic circulation (arteries, capillaries, veins) and the pulmonary circulation (arteries, capillaries, veins). Each of these components may be represented by one or multiple simple or complex models of different scales (3D, 2D, 1D, 0D). Figure 3 presents a set of possible models for the systemic and pulmonary circulation. In one embodiment, the loop of systems in the closed-loop cardiovascular system is modeled without the specific parts of the systemic arterial circulation of the third column. The order of the systemic arterial circulation is reduced to more general terms rather than specifically modeling the parts of the arterial circulation.

Alternatively, the model represents additional resolution, scale, and/or components. In yet other alternatives, the model simplifies to a reduced order, representing the cardiovascular and/or regulatory systems from a broader perspective, such as representing the heart in general with the pulmonary and systemic circulation separately.

Any model may be used. A three-dimensional (3D) model representing the anatomy, a lumped representation, or a combination of 3D and lumped may be used. In one embodiment, the closed loop cardiovascular system model is modeled as a lumped system. Due to the prohibitive computational cost of spatial blood flow models (e.g., 3D models), the closed loop model of the cardiovascular system is created as a lumped parameter model. Figure 4 shows an example "electrical" model representing the cardiovascular circulation. This closed-loop cardiovascular system model is based on the analogy between hydraulics and electricity, in the form of RLC circuits, where:

| Hydraulics | Electricity | |
|---|---|---|
| Pressure | Voltage/Potential | P |
| Flow rate | Current | Q |
| Viscosity | Resistance | R |
| Inertia | Inductance | L |
| Compliance | Capacitance | C |

In the example of Figure 4, the time-varying elastance models are used for each of the four chambers (e.g., left and right atrium and ventricle) of the heart:

$$P(t) = E(t) \cdot (V(t) - V_0) - R_s Q(t), \quad (1)$$

where E is the time-varying elastance, $V$ is the cavity volume, $V_0$ is the dead volume of the cavity, $R_s$ is a source resistance, which accounts for the dependence between the flow and the cavity pressure, and t is time. Solving for $R_s$ provides $R_s = K_s E(t)(V(t) - V_0(t))$, where $K_s$ is a constant. The cavity volume is equal to:

$$dV/\mathrm{dt} = Q_{in} - Q_{out}\ . \quad (2)$$

The lumped model also models the four valves (mitral, aortic, tricuspid, and pulmonary) of the heart. These valve models include a resistance, an inductance, and a diode. The diode is for simulating the opening and the closing of the valve based on the pressure gradient between the two sides of the valve. When the valve is closed, the flow across the valve is set to 0. When the valve is open, the following relationship holds:

$$P_{in} - P_{out} = R_{valve} \cdot Q + L_{valve} \cdot dQ/\mathrm{dt}\ , \quad (3)$$

where $P_{in}$ and $P_{out}$ represent the pressures at the inlet and the outlet of the valve, respectively. Each valve opens when $P_{in}$ becomes greater than $P_{out}$, and closes when the flow rate becomes negative.

A three-element Windkessel model is used for the systemic circulation, represented by the following relationship between instantaneous flow and pressure:

$$\frac{dP_{Ao}}{\mathrm{dt}} = R_{sys-p}\frac{dQ_{Ao}}{\mathrm{dt}} - \frac{P_{Ao} - P_{ven}}{R_{sys-d} \cdot C_{sys}} + \frac{Q_{Ao}(R_{sys-p} + R_{sys-d})}{R_{sys-d} \cdot C_{sys}}, \qquad (4)$$

where $R_{sys\text{-}p}$ and $R_{sys\text{-}d}$ are the proximal and distal resistances respectively, $C_{sys}$ is the compliance, and $P_{ven}$ is the venous pressure. A two-element Windkessel model is used for the systemic venous circulation:

$$\frac{dP_{ven}}{\mathrm{dt}} = \frac{Q_{ven}}{C_{sysVen}} - \frac{P_{ven} - P_{RA}}{R_{sysVen} \cdot C_{sysVen}}. \qquad (5)$$

The same models as represented in equations 4 and 5 are used for the pulmonary circulation. Different models may be used for any of the circulation and/or heart models. Together, the models are a lumped model of the closed-loop cardiovascular system model.

Figure 5 shows another example closed-loop cardiovascular system model. The model is a combination of a lumped model and a three-dimensional model. Part or all of the heart and/or circulation is modeled in 3D. In the example of Figure 5, the left and right ventricles are modeled in 3D while the reminder of the heart and circulation are modeled with lumped parameters. A diagnostically or therapeutically useful metric is computed using the lumped and 3D combined representation of the closed loop cardiovascular system.

If the focus of the model of the cardiovascular system lies on a specific part of the circulation, a more detailed model may be coupled to the rest of the cardiovascular system. In the example of Figure 5, the more detailed model is the 3D model, but a lumped model with additional parameters may instead be used. In the example of Figure 5, the focus is on the ventricles, but may be on other parts. The 3D model has any parameters, such as a mesh for the tissue boundary, parameters defining the physical operation of the valves and/or heart muscle, electrical activation parameters, and/or other information defining the 3D model.

The three-dimensional models of the ventricles are coupled to the above described closed-loop lumped model of Figure 4. The 3D model substitutes the time-varying elastance model of the lumped parameter model. The coupling is based on the exchange of pressure and flow rate (volume) information, but other values may be exchanged at the interface of the models. In one configuration, the following information may be exchanged: the 3D model provides the ventricular pressure and the rate of volume change at the current time step $(P_v^n, (dV/\ dt)^n)$; and the lumped model provides pressure in the atrium and in the aorta at the next time step $(P_A^{n+1}, P_{Ao}^{n+1})$. The 3D and lumped models interactions are used as boundary conditions for implementing the modeling.

The parameters of the models are personalized in act 24. Different patients vary in cardiovascular operation. To capture this variation, the values of the parameters vary or are different for different patients. While some parameters may be assigned average, median, pre-determined, set, or other values, one or more of the parameters have a value based on the information from the patient.

Acts 26-28 show example personalization of the model. These acts represent personalizing different types of models. Any one or more parameters in a given type may be personalized. One or more types of models may not be personalized, such as using a generic lumped model with personalization of one or more parameters of the 3D model. Additional, different, or fewer acts personalizing the parameters may be used, such as analyzing sensitivity to determine which parameters to personalize for a given patient.

In act 26, the model of part or all of the heart is customized. The processor determines an anatomical function model and a hemodynamic model personalized to the patient as part of a 3D model. Electrical activation and hemodynamic load from the models are provided to a biomechanical model to personalize the 3D biomechanical model. The biomechanical model includes active and passive components. Since both function from a cellular level and anatomy from an organ level are used, the model is a multi-scale model.

In one embodiment, the heart portion 40 of the closed-loop cardiovascular system model of Figure 4 or 5 is personalized using a patient-specific computational model of heart function. Anatomical, functional and hemodynamics data are integrated to estimate a generative model of cardiac electromechanics.

To couple the heart portion 40 with the circulation portion 42, the values of any of the solution variables (e.g. pressure, flow rate, velocities, or others) are exchanged at each time step. The coupling may be performed implicitly or explicitly. For example, the coupling is performed as follows: the whole-body-circulation portion 42 reads both pressure and flow values from the heart portion 40, while the heart portion 40 reads pressure values in the arterial sinus and in the venous system from the circulation portion 42.

The overall function of the enhanced heart model portion 40 is derived from imaging and clinical data of a patient for personalization. Any heart model may be used.

In one embodiment, a unified ultrasound heart model is enhanced with myocardium fiber information. The fiber information is derived either from a generative, rule-based model or from diffusion tensor imaging (DTI). A computationally efficient model of cardiac electrophysiology is used. From a 3D mesh representing the anatomy of the heart, cardiac potentials are calculated over time according to lattice-Boltzmann electrophysiology (LBM-EP). LBM-EP relies on the lattice-Boltzmann method to solve an anisotropic mono-domain equation of cardiac EP. Any cellular model may be employed. In one approach, the Mitchell-Schaeffer model is used. Tissue anisotropy is considered, in which electrical activation is faster along the myocardium fibers than across. The model is coupled to a torso model for the computation of ECGs. The measured ECGs are used to personalize the LBM-EP. The scan data for the patient is used to create a personalized 3D mesh.

The embodiment also includes a model of cardiac hemodynamics. A lumped parameter model (e.g., one pressure value is calculated for the entire cardiac chamber) controls the cardiac phases according to arterial pressures (e.g., calculated using a 3-element Windkessel model) and atrial pressures (e.g., calculated using a lumped model of atrial contraction). The cuff-pressure is used to personalize the model of cardiac hemodynamics. In another embodiment, a full 3D computational fluid dynamics solver is employed with fluid-structure interactions based on the cuff pressure and scan data.

The LBM-EP and cardiac hemodynamics are used together for the computationally efficient model of cardiac electro-mechanics. A biomechanical model of the heart is employed to calculate the pumping function resulting from the electrical activation and the hemodynamics load calculated in the EP and cardiac hemodynamic models.

For the biomechanical model, two components are used: a passive component to capture the orthotropic nature of myocardium tissue (myocardium fibers and fiber sheets) and an active component that calculates the stress created by a myocyte during contraction. Each component is controlled by a set of parameters, which may vary spatially. For example, the passive elasticity component may be any model that provides a stress-strain dependency, for example linear elasticity models or, more accurate, nonlinear models like the Hyper-elastic Orthotropic Tissue Model proposed by Holzapfel and Ogden (HO). Method specific parameters, like Young's moduli, Poisson ratios, shear moduli for linear elastic models, or parameters specific to the HO energy function, are either set based on population averages, or estimated from the scan data using inverse modeling or machine learning. The active elasticity component may be either a biophysical model, a multi-scale phenomenological model or a lumped model, any of them being dependent on tunable parameters. Examples of such parameters are the strength of the active contraction, the rates of contraction and relaxation, the time interval between cell depolarization and initiation of the contraction, as well as the transmembrane potential at which a cell is depolarized. Active model parameters are also set on a population average base or are estimated from the scan data using inverse modeling or machine learning. The calculated parameters are applied directly or with no change in the closed-loop model. Alternatively, the calculated parameters are altered to couple with the closed-loop model.

Estimation of cardiac electrophysiology parameters (e.g., electrical conductivity and action potential duration) may be further refined by leveraging strain maps of the heart computed from ultrasound. In a first approach, lines of blocks are identified from the strain map as the direction of fibers and used as prior knowledge to the EP estimation. In a second approach, the local mechanical activation speed is calculated from motion, and the resulting map is used as first estimate of electrical conductivity, which is then refined using global ECG features.

The strain maps may be used to refine estimation of cardiac biomechanical parameters (e.g., active stress and tissue stiffness). A cost function includes a difference between the calculated and computed 3D strains. Owing to the 3D acquisition, the strain tensors are directly compared in an embodiment using a log Euclidean framework. In this way, regional or localized estimates are obtained. Furthermore, coupling the personalized EP model and the image-derived motion and strain maps, the location and extent of any scar may be inferred. Should invasive endocardial mapping or body surface mapping be available, scar border zone areas may be identified as akinetic areas (e.g., as quantified on the strain maps) with electrical activity.

For estimation of cardiac hemodynamics (e.g., arterial Windkessel parameters and atrial parameters), color Doppler, pulse-wave or continuous-wave Doppler ultrasound is used as the flow directly provides pressure gradients and flow through valves, which are inputs to the closed-loop model.

These enhancements in the personalization procedure are made possible by the unified ultrasound heart model, which incorporates anatomical, dynamic, and functional information in one system. Other personalization may be used. At the end of the process, a virtual representation of a specific patient's heart is obtained, whether in a lumped model, a 3D model, or a combination of lumped and 3D model. This model for the heart portion 40 may be probed to test different therapy outcomes.

The parameters of the lumped model for the heart and/or circulation are personalized in act 28. The values of the lumped model are fit to the measured data, such as the scan data, the ECG data, and/or the pressure data. The values of the parameters resulting in the lumped model calculation of a same metric or metrics as measured are used. Alternatively, the measurements from the patient are used to estimate values for one or more parameters directly.

In addition to or as an alternative to personalizing parameters of the lumped model, the lumped model for the circulation portion 42 of Figure 4 may be personalized using additional parameters. For example, the cardiovascular systemic impedance and the cardiovascular pulmonary impedance personalized to the patient are determined with inductance of arterial sinuses, aortic arteries, and/or pulmonary arteries, and/or with resistances of the arterial tree. Figure 6 shows a lumped model for systemic/pulmonary circulation that introduces further possible control parameters in the form of inductances at the level of arterial sinuses and/or aortic/pulmonary arteries, and/or individual resistances at various levels of the arterial tree. A possible model for the pulmonary and systemic circulation is governed by the following equations, represented with subscripts for the systemic case:

$$\frac{dP_{as}}{dt} = \frac{Q_{vent} - Q_{as}}{C_{as}} \text{ and flow rate } \frac{dQ_{as}}{dt} = \frac{P_{as} - P_{at} - Ras * Qas}{L_{as}} \qquad (6)$$

while for the distal part of the system:

$$\frac{dP_{at}}{dt} = \frac{Q_{as} - Q_{at}}{C_{at}} \text{ and flow rate } \frac{dQ_{at}}{dt} = \frac{P_{at} - P_{vn} - (Rat + Rar + Rcp) * Qat}{L_{as}} \qquad (7)$$

and the venous circulation equations are:

$$\frac{dP_{vn}}{dt} = \frac{Q_{at} - Q_{vn}}{C_{at}} \text{ and flow } Q_{vn} = \frac{P_{vn} - P_{ra}}{R_{vn}}. \qquad (8)$$

Other lumped models may be used.

To further personalize the heart portion 40, the time-varying flow rate for the heart and the pressure variation for the heart are calculated with the lumped model including KG diaphragm dynamics. The model of the heart is enhanced to model the influence of the KG diaphragm on the flow. The KG diaphragm is a soft tissue, including the annulus fibrosus and the four heart valves. The KG diaphragm undergoes periodic displacement into the atrioventricular chambers under the combined action of several forces, including: the pressure force due to the pressure difference across the valves and surrounding tissue, the tissue strain forces from both the atrium and the ventricle sides that act on the base of the annulus fibrous, the frictional force from blood flow, and the elastic force due to the elasticity of the KG diaphragm. The KG diaphragm dynamics depends on the balance of all these forces. Its dynamics is modeled for each of the two cardiac sides separately.

In the systolic phase, the KG diaphragm moves into the ventricular chamber, and in the end diastolic phase, the KG diaphragm moves into the atrium due to the atrial contraction. The total displacement along the long axis of the heart may be about 2-3 cm. One may model the KG diaphragm dynamics using a lumped parameter model:

$$\mathrm{M_{dia}} \frac{\mathrm{d^2 l}}{\mathrm{dt^2}} + \mathrm{D_{dia}} \frac{\mathrm{dl}}{\mathrm{dt}} + \mathrm{K_{dia}} * \mathrm{l} = \mathrm{F_{atr}} - \mathrm{F_{vent}} + (\mathrm{P_{vent}} - \mathrm{P_{atr}}) \cdot \mathrm{A_{dia}} \qquad (9)$$

Equation (9) prescribes that the changes in the displacement of the annulus fibrosus l are governed by internal (left side of the equation) and external (right side of the equation) contributions. The internal contributions are due to inertia $\mathrm{M_{dia}} \frac{\mathrm{d^2 l}}{\mathrm{dt^2}}$ (where $M_{dia}$ is the diaphragm tissue mass), damping forces $\mathrm{D_{dia}} \frac{\mathrm{dl}}{\mathrm{dt}}$ (with $D_{dia}$ a damping multiplication parameter), and elastic forces $K_{dia} * 1$ (where $K_{dia}$ is the diaphragm elasticity). These are balanced by external forces due to cavity strain on either side of the diaphragm ($F_{atr}$ or $F_{vent}$) which may be modeled to depend on the atrial or ventricular elastance (e.g. $F_{vent} = K_{vent} * e_{vent}$ with $e_{vent}$ being the elastance function, and similarly for atrium), and forces due to pressure difference across the diaphragm $(P_{vent} - P_{atr}) \cdot A_{dia}$, where $A_{dia}$ is the area of the diaphragm.

The motion of the KG diaphragm redefines the location of the atrioventricular boundary at each time instant and introduces volume changes to the two left chambers. The location and volume changes are represented as:

$$V_{vent} = V_{vent} + A_{dia} \cdot l \text{ and } V_{atr} = V_{atr} - A_{dia} \cdot l. \qquad (10)$$

where the ventricular ($V_{vent}$) and the atrial ($V_{atr}$) volumes are adjusted by the change in volume due to the diaphragm motion, given by $A_{dia} \cdot l$. The diaphragm movement depends on a series of parameters: the elastance of the chambers, geometric parameters (like the sectional area of the annulus fibrosus) and other coefficients (Kst, Kf, etc.). The personalization of the elastance is performed as described before (i.e., by matching model outputs and patient-specific measurements). The geometric parameters are personalized from the medical images. The coefficients may also be personalized from medical images acquired at different time points of a cardiac cycle, providing information regarding the timing and the extent of the diaphragm movement.

Referring to Figure 3, the regulatory system may be modeled with a baroreflex system model coupled to the closed loop cardiovascular system model. To model various steady states and transient states of a patient, one or more of the regulatory systems displayed in Figure 3 are modeled. The baroreflex system is one of the most complex systemic regulatory systems. The input is the mean arterial pressure at the aortic arch and the carotid sinus. These inputs may be provided by the cardiovascular system model. The baroreflex system is composed of three main parts: the afferent part (the baroreceptors), the central nervous system, and the efferent part (efferent pathways). The baroreflex system controls the heart rate, the contractility of the left and right ventricle, the systemic arterial resistance, the venous compliance, and the venous unstressed volume. The modeling of the baroreflex system is applicable, for example, when simulating (acute) hemorrhage or heart pacing.

The coupling of the cardiovascular system model with the regulatory systems is performed as exchange of information between the models. In one embodiment, the whole body circulation model outputs certain hemodynamic variables (e.g. pressure and flow rate). These outputs are input to the model of the regulatory system. The model for the regulatory system in turn modifies the parameter values of the whole-body circulation model. The model of a regulatory system may be called only once at the end of a heart cycle, using cycle averaged hemodynamic quantities as input information, or at each time step, using instantaneous hemodynamic quantities as input information.

While the baroreflex system affects several parameters of the whole body circulation model, the other regulatory systems usually modify mainly one parameter. For example, the cerebral, renal, and coronary autoregulation systems mainly affect the microvascular resistance of the corresponding organs. Differential equations may be used. For example for the coronary autoregulation, $R_{micro} = f(Q)$ where $R_{micro}$ is the microvascular resistance and Q is the cycle-averaged coronary flow rate.

To compute patient-specific hemodynamics, the circulatory and regulatory models are personalized. Any approach to personalization may be used. A value of a model parameter is set to or based on a measured value (e.g., formula relates one or more measured value to the value for the parameter). In another approach, various values of parameters are tested or solved to match a model-based calculation to a measurement.

Figure 7 shows an example workflow for fully automatic model personalization. A processor performs the acts after user selection of metrics. In act 72, the metrics of interest are defined by the user or the processor. The metrics of interest are the diagnostically or therapeutically relevant information. For example, the pressure-volume (PV) loop of the left and/or right ventricle, the stroke work, the arterial ventricular coupling (i.e., arterial elastance divided by left ventricular end-systolic elastance), the isochrone volume foot, and/or the myocardial strains are metrics that may assist a physician in diagnosing or treating a cardiovascular condition.

In act 70, patient-specific measures for the metrics of interest are extracted. The obtained data is used to obtain the measures. Any measures may be used. For example, the measures may be non-invasive measurements (e.g., cuff-based pressures, heart rate, echocardiography based measures (volume, blood velocity, and/or arterial dimensions) and/or imaging based measures (e.g., flow rate, velocity, movement of the arterial walls). As another example, the measures may be invasive measurements (e.g., invasive pressure, flow, or resistance measurements at any location in the cardiovascular system).

In act 74, the processor performs a sensitivity analysis of parameters of the multi-scale whole body circulation model for the patient. The sensitivity analysis identifies the parameters of the models that affect the metrics of interest. A threshold may be used to determine the parameters that sufficiently influence the metric or metrics. For sensitivity analysis, global sensitivity analysis and uncertainty quantification are performed. Any sensitivity analysis may be used, such as the stochastic collocation method or polynomial chaos expansion. As an alternative to sensitivity analysis, predetermined parameters or user-selected parameters are used.

Once the parameters with the highest influence on the metrics of interest have been identified, the processor personalizes the parameters in act 76 based on the patient-specific measures. The selected sub-set of the parameters from act 74 is personalized. Any personalization may be used, such as using a measure directly as the value of the parameter, calculating the value of the parameter from measures, and/or solving for the values of parameters using the measures.

In one embodiment, the processor solves for the parameters based on a difference between measured and modeled values. The personalization may include running a forward model multiple times in act 78 until certain objectives in the model outputs are met, such as minimization of differences between model-calculated values and measured values. Furthermore, simplified models may be used during this process to speed-up the iterations required for finalizing the personalization. For example, a reduced order model using fewer parameters (e.g., more lumping) is used to solve for the values of the parameters. Alternatively, the full-scale model (e.g., lumped, 3D, or lumped+3D) is used to solve for the values of the parameters based on the measurements from the specific patient.

Once a first personalization is performed, the sensitivity analysis and uncertainty quantification may be rerun to more accurately determine parameters for personalization for the current patient. Rather than performing the sensitivity analysis for the model in general, the sensitivity analysis is performed for the model as tuned or personalized to the specific patient. This approach is represented by the feedback arrows from act 78 to acts 74 and 76.

In one example, personalization is provided for computing patient-specific left ventricular PV loops using a lumped parameter model. The model personalization framework includes two sequential steps. First, a series of parameters are computed directly, and, next, an optimization-based calibration method is employed to estimate the values of the remaining parameters, ensuring that the personalized computations match the measurements. The input parameters are cuff-based systolic and diastolic pressure (SBP and DBP), the heart rate (HR), and echocardiography based ejection fraction (EF) and end-diastolic and systolic volume (EDV and ESV).

During the first step of the parameter estimation framework, the mean arterial pressure (MAP) is determined:

$$MAP = DBP + \left[1/3 + \left(HR \cdot 0.0012\right)\right] \cdot (SBP - DBP) \,. \tag{11}$$

Then, the end-systolic volume is computed:

$$ESV = EDV \cdot \left(1 - EF\right)/100 \,. \tag{12}$$

Next, the stroke volume is determined:

$$SV = EDV - ESV \,, \tag{13}$$

and the average aortic flow rate is computed:

$$\overline{Q_{Ao}} = SV \cdot 60/HR \,. \tag{14}$$

Finally, the total systemic resistance, as well as the proximal and distal components, are determined:

$$\begin{gathered} R_{sys-t} = \left(MAP - P_v\right)/\overline{Q_{Ao}}\,, \\ R_{sys-p} = \rho \cdot R_{sys-t}; \quad R_{sys-d} = \left(1-\rho\right)\cdot R_{sys-t}\,, \end{gathered} \tag{15}$$

where p is the proximal resistance fraction. Other functions may be used.

During the second step of the parameter estimation framework, an optimization-based calibration method is employed to estimate the maximum elastance of the left ventricle model, $E_{max-LV}$, the dead volume of the left ventricle, $V_{0-LV}$, and the compliance of the systemic Windkessel model, $C_{sys}$. The parameter estimation problem is formulated as a numerical optimization problem, the goal of which is to find a set of parameter values for which a set of objectives are met. Since the number of parameters to be estimated is set equal to the number of objectives, the parameter estimation problem becomes a problem of finding the root for a system of nonlinear equations. To solve the system of equations, the dogleg trust region method is used. The objectives of the parameter estimation method are formulated based on the systolic and diastolic pressures, and the ejection fraction, leading to the system of nonlinear equations:

$$\boldsymbol{r}\begin{pmatrix} E_{max-LV} \\ V_{0-LV} \\ C_{sys} \end{pmatrix} = \begin{Bmatrix} (SBP)_{comp} - (SBP)_{ref} \\ (DBP)_{comp} - (DBP)_{ref} \\ (EF)_{comp} - (EF)_{ref} \end{Bmatrix} = \begin{Bmatrix} 0 \\ 0 \\ 0 \end{Bmatrix}, \tag{16}$$

where, ***r(x)*** is a vector function, called in the following objective function, and x is the vector of the unknowns (i.e., the parameters to be estimated). Each component of the objective function is formulated as the difference between the computed value of a quantity - $(\bullet)_{comp}$ (determined using the lumped parameter model) and its reference value - $(\bullet)_{ref}$ (determined through measurement in act 70). To evaluate the objective function for a given set of parameter values, the lumped parameter model is run only once or multiple times.

A similar personalization approach may be applied for the model configuration in Figure 5. Different parameters may be personalized for the 3D ventricle models (e.g. maximum active force and passive biomechanical tissue properties). Besides the optimization-based method of Figure 7, other methods may be used for fully automated iterative calibration. Fitting-based or surrogate model approaches may be used. For any of these methods, the number of parameters to be estimated may be either smaller, equal, or larger than the number of objectives or measures from the patient.

The cuff pressures (e.g., measured at the arm) used for personalization may be further adapted before being used as objectives in the parameter estimation procedures. A transfer function estimates the central arterial pressure from the measured cuff pressure.

Generally the parameter estimation problem may be formulated as:

$$\boldsymbol{r}(\boldsymbol{p}) = \{\boldsymbol{o}_{comp} - \boldsymbol{o}_{ref}\} = \{0\} \tag{17}$$

where p is the vector of parameters, and o is the vector of objectives ($\boldsymbol{o}_{comp}$ is the vector of objectives obtained from the forward model, and $\boldsymbol{o}_{ref}$ is the vector of objective reference values measured from the patient).

Direct personalization may also be performed specifically for subparts of the cardiovascular model. For example, a specialized lumped parameter valve model may be used, given by the following equation:

$$\Delta p = Rq + Bq|q| + L\frac{dq}{dt} \tag{18}$$

where *R,* B and L are three parameters given by the blood properties and the geometry of the valve:

$$\{B, F\} = f(A_{eff,\max}, A_{eff,\min}, A_{prox}, A_{distal}, valve\ timing, \rho, \mu, etc.) \tag{19}$$

where $A_{eff,max}$ is the maximum annulus area, $A_{eff,min}$ is the minimum annulus area, $A_{prox}$ is the cross-sectional area proximal to the valve, $A_{dist}$ is the cross-sectional area distal to the valve, *valve timing* refers to the dynamics of valve closure and opening, p is the blood density, $\mu$ is the blood viscosity. This information may be extracted non-invasively using different imaging modalities (e.g. echocardiography).

One, a sub-set, or all parameters are personalized for a specific patient. A generic population-average value, median, or other predetermined value may suffice for some of the parameters.

Different approaches may be used in dealing with a combined lumped and 3D model. In one approach, the parameters for the lumped model configuration in Figure 4 are personalized. Then, these lumped parameter values are used for the lumped portion of the multi-scale model of Figure 5. The parameters of the 3D portion are personalized based on the personalized lumped model. In another approach, the parameters for the lumped model configuration in Figure 4 are personalized. Rather than directly using these parameters for the lumped portion of Figure 5, the personalized values of the lumped parameters are used to initialize the full-scale model of Figure 5. The personalization is then rerun for the entire model of Figure 5.

Referring again to Figure 1, the processor computes one or more metrics with the personalized multi-scale, whole-body circulation model in act 32. For example, the time-varying flow rate for the heart, pressure variation for the heart, cardiovascular systemic impedance, and cardiovascular pulmonary impedance personalized to the patient are used to compute a value of a metric. The resulting parameters adapt the model for the patient. In one embodiment represented in act 36, the personalized parameters are then ones determined from the sensitivity analysis. The adapted or patient-specific model is used to calculate the diagnostically or therapeutically useful information. The multi-scale, whole-body circulation model is run with the personalized parameters of the sub-set.

Where multiple models are provided, such as shown in Figures 2 and 3, the calculation of the metric relies on coupling or interaction between the models in act 34 (see Figure 1). Values of parameters used by one model may be calculated by another model. The exchanged values provide time varying boundary conditions for modeling part of the cardiovascular system and/or the regulatory system. For example in act 34, pressure, flow or other values are determined for regulated regions of the heart. These values for a given time are passed to one or more regulatory models. The regulatory models alter the values of these parameters and pass the altered values back to the cardiovascular model for calculation in a next time step, emulating regulation of the function of the heart.

Based on the modeling at a desired cardiac phase or over time, one or more metrics are computed. For example, a pressure-volume (PV) loop of a ventricle, a stroke workload, arterial-ventricular coupling, isochrones volume foot, and/or myocardial strain are computed from the model.

Figure 8 displays example PV results for a patient with mild regurgitation. The results from modeling are compared with measurements of the same metric from the patient. There is a close agreement between the time-varying LV and aortic pressures, time-varying LV volumes, and PV loops. Moreover, the four phases of the cardiac cycle may be clearly identified in the computed results: 1: isovolumetric contraction phase, 2: ventricular ejection phase, 3: isovolumetric relaxation phase, and 4: ventricular filling phase. The mild aortic valve regurgitation may be observed in the PV loop, where the line corresponding to the isovolumetric relaxation has a slight curvature.

In act 38 of Figure 1, the metric or metrics are indicated on a display. The metric may be a value, graph, vector field, or spatial distribution. The metric is displayed on a screen, such as displaying the PV or other values as shown in Figure 8 without comparison to measurements. Other displays of the indication may be provided, such as indicating a workflow or providing instructions based on the metric. In alternative embodiments, the metric is stored in the patient record and/or transmitted on a computer network.

Figure 9 shows one embodiment of a method for personalized whole-body circulation calculation. Machine learning is used to enhance the operation of the method of Figure 1 or other personalized whole-body circulation calculation approach. The machine learning is combined with operating a full-scale model of the cardiovascular system and operating a reduced scale model of the cardiovascular system.

The acts are performed in the order shown or another order. For example, act 82 is performed before act 80 or both are performed in parallel. Act 84 is performed in parallel with either of acts 80 or 82 in other embodiments. Figure 9 is shown for a training phase. For application of a machine-learnt classifier, the application act may occur at any time relative to running the full and reduced order models.

Additional, different, or fewer acts may be provided. For example, Figures 10 and 11 show additional acts.

In act 80, a full-scale model of the cardiovascular system is run. A processor performs the modeling of the whole-body circulation of the patient. The full-scale model is the model with the greatest number of parameters and/or variables (i.e., the model with the highest order). The full-scale model may be a lumped, 3D, or lumped + 3D model. "Full" is used as a relative term in comparison to the "reduced" scale model. The full-scale model has less simplification, more parameters, and works on a higher dimensional domain compared to the reduced scale model.

The full scale model is personalized. Alternatively, the full-scale model is without personalization and is being run in order to personalize.

In act 82, the processor runs the reduced-scale model of the whole-body circulation of the patient. The reduced scale model is personalized. Alternatively, the reduced scale model is without personalization.

In act 84, the processor applies machine training to learn a classifier or regressor to predict based on outputs of running the full and reduced scale models. The outputs are personalized parameter values and/or metrics calculated using the personalized parameter values. The outputs are used alone or with other information as an input vector for the machine learning.

Any machine learning may be used. For example, a neural network, Bayesian network, probability boosting tree, support vector machine, regression, instance-based method, regularization method, decision tree learning, kernel method, clustering method, association rule learning, dimensionality reduction, or ensemble method is used. Given many examples as training data, the machine learning learns to predict based on the input vector.

The training creates a regressor to estimate values for parameters used in the reduced order model for a given patient (see Figure 10) or to estimate a metric as a replacement for the model (see Figure 11). In the embodiment of Figure 10, the machine-trained classifier or model predicts parameters of the reduced scale model based on training using parameters provided by the reduced and full-scale models of whole-body circulation. The coefficients of the reduced scale model are adapted or set based on the outputs of the running of the full-scale model. The machine-learnt classifier then predicts the coefficients of the reduced scale model from specific patient information. The parameters of the reduced-scale, whole-body circulation model (e.g., used as the reduced order model) are predicted with the machine-trained model trained from parameters (e.g., values of parameters) provided by a full-scale, whole-body circulation model.

Figure 10 shows one embodiment of machine-learning based improvement of a physiological reduced-order model. Machine learning based approaches may be used to improve reduced-order models. A full-scale (e.g., three-dimensional) model provides higher fidelity when computing measures of interest compared to a reduced-order model. However, the execution time required for running a full-scale model may be considerably larger, so may not be appropriate for clinical settings. These full and reduced order models may refer to blood flow computations, cardiac mechanics, electrophysiology, fluid-structure interaction applications, or other aspects of whole-body circulation.

By using a machine-learning method trained based on simulations performed with the full-scale model, the reduced-order model may be improved. For example, additional terms may be added in the reduced-order model to account for the effect of properties that are not captured by the reduced-order model. Alternatively or additionally, coefficient values (i.e., values of parameters) of existing terms may also be refined using this approach.

Referring to Figure 10, a large number of input data sets (geometry, lumped parameter values, patient measurements, or other information) are generated in act 103. The database used for training the machine-learning algorithm may contain patient-specific input data sets, synthetically generated input data sets, or both.

In act 104, full-scale simulations are performed. A set of features describing the property that is not captured by the reduced-order model are extracted from input data set in act 105. A set of metrics of interest are extracted from the computational results in act 100.

In act 101, the reduced-order computations are performed and the terms/coefficients in the reduced-order model are adapted in act 102 to match the metrics of interest extracted from the full-scale model in act 100. The machine-learning algorithm is trained in act 106 to be able to predict the values of the parameters or coefficients solely from the features extracted from the input data (features of act 105 and parameters from act 102).

For example, considering the left ventricle, a popular reduced-order model is the time-varying elastance model. The source resistance $R_s$ is one coefficient whose value may be set using the workflow in Figure 10, possibly by focusing on specific pathologies, like: hypertrophy or cardiomyopathy (e.g., arrhythmogenic right ventricular cardiomyopathy, isolated ventricular non-compaction, mitochondrial myopathy, dilated cardiomyopathy, restrictive cardiomyopathy, peripartum cardiomyopathy, takotsubo cardiomyopathy, loeffler endocarditis, or others). Similarly, the minimum and/or maximum elastance, the dead volume, and any other parameters of the time-varying elastance model may be set using the workflow in Figure 10. Moreover, additional terms (e.g., constant or based on pressure, volume, and/or flow rate) may be added in the equation. The improvement of the reduced-order model may further be targeted at different physiological states of the patient, such as rest, exercise, pre-prandial or postprandial, drug-induced hyperemia, heart pacing, hemorrhage, or another state.

The workflow of Figure 10 refers to the case when both the full-scale and the reduced-order models are physiological models and the machine learning-based method is used to improve the reduced-order model. In an alternative approach, a learning based technique may be used to derive a data-driven reduced-order representation of a physiological model, which in turn may be full-scale or reduced-scale. The model is created using mapping, such as a regression relating inputs to outputs. For example, a data-driven model reduction of a cardiac electrophysiology model is provided. As another example, a data-driven model reduction of a cardiac myofilament model is provided.

Once trained, input data is generated in act 109 from a specific patient. The features are extracted from the input data in act 110. The same features as extracted in act 105 are used. These features are applied to the trained classifier in act 107. The classifier outputs the coefficients or terms (e.g., values for the parameters) to be used by the reduced order model in act 108. The reduced order model is run to calculate the metric or metrics of interest based on the personalized parameters predicted by the machine-learnt classifier.

In another embodiment of Figure 9, the machine training trains as a forward model with features extracted from the multi-scale, whole-body circulation model. This classifier is used to compute metrics or predict the output of the reduced scale model based on personalization information from the patient. The metric of interest, such as the pressure-volume loop, is output by the machine-learnt classifier.

Figure 11 shows an example flow chart for a machine learning-based forward model with features extracted from a reduced-order model. Machine learning is used to derive fast data-driven models of physiological models used in blood flow computations, cardiac mechanics, electrophysiology, and/or fluid-structure interaction applications. These machine-learning methods use the input parameters of the reduced-order model and/or other input data from the patient as features and the outputs of the full-scale physiological model as metrics of interest that represent the target values (i.e., ground truth) during training. The classifier is trained to predict the output of the reduced order model, but includes knowledge gained from the full-scale model.

For training, input data sets for many patients are acquired in act 112. The same and/or different features are extracted from the patient data in act 111 and provided in act 114 as output from running a reduced order model in act 113 personalized to the patients. During training, the full-scale model is used in act 117 to determine the target values (e.g., metrics of interest) for training the data-driven model in act 115. The input data for the training are the values for features extracted in act 111, the values for the features extracted in act 114, and the values for the metric of interest in act 117. Measured values of the metric of interest may be used instead or in addition to the values from act 117. Performing act 115 provides a machine-learnt classifier that predicts the metric of interest given an input of values of features from the patient data and from the reduced order model.

In one example, when predicting trans-coarctation pressure drops, three-dimensional blood flow computations may be used for an accurate estimation. These computations of the full-scale model however have execution times of several hours. Hence, an alternative is to use a reduced-order computation based on lumped parameter models or one-dimensional models, whose execution times are at least two orders of magnitude lower. The results from the reduced order model may be used as features for a machine-learning algorithm. Other or the same patient-specific features or geometric features extracted directly from the input data may also be part of the input vector for the machine learning and application of the learnt classifier.

For application of the classifier in act 116, patient-specific data is acquired in act 118. Based on this patient-specific data, features (e.g., measurements or values derived from measurements) are calculated in act 119 with a personalized reduced-order model and are extracted from the patient data in act 120. The machine-learnt classifier outputs the metric of interest for the patient based on these input features.

This workflow of Figure 11 may alternatively be an approach for data-driven improvement of results provided by a reduced-order model. The database used for the training phase may contain patient-specific input data sets, synthetically generated input data sets, or both.

Depending on the available patient-specific metrics, certain components of the whole body circulation model may be represented by spatial models instead of lumped parameter models. For example, if the cuff-based pressure measurements at the arm of the patient are available, the arterial circulation between the ascending aorta and the measurement location may be represented by a one-dimensional model in order to capture the pressure and flow rate wave propagation effects between the two locations. Thus, the afterload of the ventricle is represented with higher fidelity in a 3D model.

Figure 12 shows a system for personalized whole-body circulation calculation. The system includes a medical imaging system 11, a processor 12, a pressure cuff 13, a memory 14, an EKG sensor 15, and a display 16. The processor 12 and the memory 14 are shown separate from the medical imaging system 11, such associated with being a computer or workstation apart from the medical imaging system 11. In other embodiments, the processor 12 and/or memory 14 are part of the medical imaging system 11. In alternative embodiments, the system is a workstation, computer, or server for computing values of metrics from data acquired by a separate system in real-time or using previously acquired patient-specific data stored in the memory 14. For example, the medical imaging system 11 is provided for acquiring data representing a volume, and a separate database, server, workstation, and/or computer is provided for personalizing and computing.

Additional, different, or fewer components may be used. For example, other sensors are used to gather patient-specific data. The pressure cuff 13 and/or EKG sensor 15 may or may not be used in other embodiments.

The computing components, devices, or machines of the system, such as the medical imaging system 11 and/or the processor 12 are configured by hardware, software, and/or design to perform calculations or other acts. The computing components operate independently or in conjunction with each other to perform any given act, such as the acts of Figures 1, 7, or 9-11. The acts are performed by one of the computer components, another of the computing components, or a combination of the computing components. Other components may be used or controlled by the computing components to scan or perform other functions.

The medical imaging system 11 is any now known or later developed modality for scanning a patient. The medical imaging system 11 scans the patient for a vessel region. For example, a C-arm x-ray system (e.g., DynaCT from Siemens), CT like system, or CT system is used. Other modalities include MR, x-ray, angiography, fluoroscopy, PET, SPECT, or ultrasound. The medical imaging system 11 is configured to acquire the medical imaging data representing part or all of the heart. Data representing one or more vessels may be acquired. The data is acquired by scanning the patient using transmission by the scanner and/or by receiving signals from the patient. The type or mode of scanning may result in receiving data of just part of the cardiovascular system. Alternatively, data of a volume region is received and the vessel information is segmented from information of other anatomy.

The pressure cuff 13 is an automated or manual pressure detector. The cuff 13 is adapted for sensing pressure on an arm of the patient, but may sense pressure at other locations. In alternative embodiments, other pressure sensors may be used, such as a pressure sensor on a catheter inserted within the patient.

The EKG sensor 15 is a plurality of electrodes connected with a circuit or processor. An EKG waveform, heart rate, and/or phase designators sensed from the electrical signals are output by the EKG sensor 15.

The memory 14 is a buffer, cache, RAM, removable media, hard drive, magnetic, optical, database, or other now known or later developed memory. The memory 14 is a single device or group of two or more devices. The memory 14 is within the system 11, part of a computer with the processor 12, or is outside or remote from other components.

The memory 14 stores the models, values of parameters, patient data, and/or other information. The memory 14 stores data resulting from the processes described herein, such as storing the constants, initial values, personalized values, computed metrics, or other properties.

The memory 14 is additionally or alternatively a non-transitory computer readable storage medium with processing instructions. The memory 14 stores data representing instructions executable by the programmed processor 12 for personalized whole-body circulation calculation. The instructions for implementing the processes, methods and/or techniques discussed herein are provided on computer-readable storage media or memories, such as a cache, buffer, RAM, removable media, hard drive or other computer readable storage media. Computer readable storage media include various types of volatile and nonvolatile storage media. The functions, acts or tasks illustrated in the figures or described herein are executed in response to one or more sets of instructions stored in or on computer readable storage media. The functions, acts or tasks are independent of the particular type of instructions set, storage media, processor or processing strategy and may be performed by software, hardware, integrated circuits, firmware, micro code and the like, operating alone or in combination. Likewise, processing strategies may include multiprocessing, multitasking, parallel processing and the like. In one embodiment, the instructions are stored on a removable media device for reading by local or remote systems. In other embodiments, the instructions are stored in a remote location for transfer through a computer network or over telephone lines. In yet other embodiments, the instructions are stored within a given computer, CPU, GPU, or system.

The image processor 12 is a general processor, digital signal processor, three-dimensional data processor, graphics processing unit, application specific integrated circuit, field programmable gate array, digital circuit, analog circuit, combinations thereof, or other now known or later developed device for modeling from medical data. The image processor 12 is a single device, a plurality of devices, or a network. For more than one device, parallel or sequential division of processing may be used. Different devices making up the image processor 12 may perform different functions, such as personalizing by one device and computation of a metric by another device. In one embodiment, the image processor 12 is a control processor or other processor of the medical imaging system 11. The processor 12 operates pursuant to stored instructions to perform various acts described herein.

The image processor 12 is configured to personalize. One or more parameters of a closed-loop cardiovascular model are personalized. The values making the model better represent a specific patient are calculated from measurements or other information for that patient. The image processor 12 is configured to compute a metric. The personalized model is used to determine a value for a metric of interest.

In one embodiment, the image processor 12 is configured to apply a machine-trained classifier. The classifier is applied for a given patient. A scan and/or other information are gathered for that patient. That data and machine-trained classifier are used by the processor 12 to determine a metric and/or to personalize a model. The classifier was trained based on a lumped model, a three-dimensional model, or a combination lumped and three-dimensional model and based on a reduced order model.

The display 16 is a CRT, LCD, plasma, projector, printer, or other output device for showing an image. The display 16 displays the quantity or quantities calculated using the personalized model. The quantities may be displayed in a chart, graph, and/or on an image.

While the invention has been described above by reference to various embodiments, it should be understood that many changes and modifications can be made without departing from the scope of the invention. It is therefore intended that the foregoing detailed description be regarded as illustrative rather than limiting, and that it be understood that it is the following claims, that are intended to define the scope of this invention.

## Claims

1. A method for personalized whole-body circulation calculation, the method comprising:

   capturing cardiovascular spatial data of a patient with a medical scanner;
   capturing cardiac electrophysiology data of the patient with a cardiac electrophysiology sensor;
   capturing pressure data of the patient with a pressure sensor;
   measuring a cardiac hemodynamic parameter from the cardiovascular spatial data;
   determining time-varying flow rate for the heart, pressure variation for the heart, cardiovascular systemic impedance, and cardiovascular pulmonary impedance personalized to the patient from the cardiovascular spatial data, the ECG data, and the pressure data;
   computing a metric with a multi-scale whole-body circulation model as a function of the time-varying flow rate for the heart, pressure variation for the heart, cardiovascular systemic impedance, and cardiovascular pulmonary impedance personalized to the patient; wherein the multi-scale whole-body circulation model comprises a cardiovascular system model coupled to one or more models representing the cardiovascular regulatory systems, and a bidirectional exchange of information between the systems leads to a continuous adaptation of the cardiovascular activity and operation, wherein the flow rate or volume change and pressure data from the cardiovascular system model are provided to the one or more regulatory system models and the one or more regulatory system models return adapted or altered values for use in the cardiovascular system model, and
   indicating the metric on a display for the patient.

2. The method of claim 1 wherein capturing the cardiovascular spatial data comprises capturing ultrasound data of the heart with the medical scanner comprising an ultrasound scanner.

3. The method of claim 1 further comprising segmenting the cardiovascular spatial data for a heart of the patient in at least two phases of a cardiac cycle.

4. The method of claim 1 wherein the multi-scale whole body circulation model includes a combination of a lumped model and a three-dimensional model of at least part of the heart, and wherein determining comprises determining with an anatomical model, a hemodynamic model, an electrophysiology model, and a biomechanical model personalized to the patient.

5. The method of claim 4 wherein determining with the biomechanical model comprises determining with active and passive components of the biomechanical model, the active component controlled by the electrophysiology model.

6. The method of claim 1 wherein determining the cardiovascular systemic impedance and the cardiovascular pulmonary impedance personalized to the patient comprises determining with inductance of arterial sinuses, aortic arteries, and/or pulmonary arteries, and/or determining with resistances of the arterial tree.

7. The method of claim 1 wherein determining the time-varying flow rate for the heart and the pressure variation for the heart comprises determining is a model of the heart valve dynamics.

8. The method of claim 1 wherein said cardiovascular system model is a closed loop cardiovascular system model.

9. The method of claim 8 further comprising altering parameters of the closed loop cardiovascular system model based on one from said one or more regulatory system models.

10. The method of claim 9 wherein altering comprises altering with the one from said one or more regulatory system models comprising a baroreflex system model coupled to the closed loop cardiovascular system model.

11. The method of claim 1 wherein computing the metric comprises computing a pressure-volume loop of a ventricle, a stroke workload, arterial-ventricular coupling, isochrones volume foot, and/or myocardial strain.

12. The method of claim 1 further comprising:

performing a sensitivity analysis of parameters of the multi-scale whole body circulation model for the patient;
personalizing a sub-set of the parameters selected based on the sensitivity analysis; and
running a forward model of the multi-scale whole body circulation model with the personalized parameters of the sub-set.

13. The method of claim 12 wherein personalizing comprise solving for the parameters based on a difference between measured and modeled values.

**Patentansprüche**

1. Verfahren zur personalisierten Ganzkörper-Kreislaufberechnung, wobei das Verfahren umfasst:

Erfassen von kardiovaskulären Raumdaten eines Patienten mit einem medizinischen Scanner;
Erfassen von kardialen elektrophysiologischen Daten des Patienten mit einem kardialen elektrophysiologischen Sensor;
Erfassen von Druckdaten des Patienten mit einem Drucksensor;
Messen eines kardialen hämodynamischen Parameters aus den kardiovaskulären Raumdaten;
Bestimmen einer zeitvariablen Flussrate für das Herz, einer Druckvariation für das Herz, einer kardiovaskulären systemischen Impedanz und einer kardiovaskulären pulmonalen Impedanz, die für den Patienten aus den kardiovaskulären Raumdaten, den ECG-Daten und den Druckdaten personalisiert sind;
Berechnen einer Metrik mit einem mehrskaligen Ganzkörper-Kreislaufmodell als Funktion der zeitvariablen Flussrate für das Herz, der Druckvariation für das Herz, der kardiovaskulären systemischen Impedanz und der kardiovaskulären pulmonalen Impedanz, die für den Patienten personalisiert sind;

wobei das mehrskalige Ganzkörper-Kreislaufmodell ein kardiovaskuläres Systemmodell umfasst, das mit einem oder mehreren Modellen gekoppelt ist, die die kardiovaskulären Regulierungssysteme darstellen, und ein bidirektionaler Informationsaustausch zwischen den Systemen zu einer kontinuierlichen Anpassung der kardiovaskulären Aktivität und des Betriebs führt, wobei die Flussrate oder Volumenänderung und Druckdaten aus dem kardiovaskulären Systemmodell dem einen oder mehreren Regulierungssystemmodellen bereitgestellt werden und das eine oder die mehreren Regulierungssystemmodelle angepasste oder geänderte Werte zur Verwendung im kardiovaskulären Systemmodell zurückgeben und die Metrik auf einem Display für den Patienten anzeigen.

**2.** Verfahren nach Anspruch 1, wobei das Erfassen der kardiovaskulären Raumdaten ein Erfassen von Ultraschalldaten des Herzens umfasst, wobei der medizinische Scanner einen Ultraschallscanner umfasst.

**3.** Verfahren nach Anspruch 1, ferner umfassend ein Segmentieren der kardiovaskulären Raumdaten für ein Herz des Patienten in mindestens zwei Phasen eines kardialen Zyklus.

**4.** Verfahren nach Anspruch 1, wobei das mehrskalige Ganzkörper-Kreislaufmodell eine Kombination aus einem Lumped-Modell und einem dreidimensionalen Modell mindestens eines Teils des Herzens umfasst, und wobei das Bestimmen ein Bestimmen mit einem anatomischen Modell, einem hämodynamischen Modell, einem elektrophysiologischen Modell und einem biomechanischen Modell umfasst, die für den Patienten personalisiert sind.

**5.** Verfahren nach Anspruch 4, wobei das Bestimmen mit dem biomechanischen Modell ein Bestimmen mit aktiven und passiven Komponenten des biomechanischen Modells umfasst, wobei die aktive Komponente durch das elektrophysiologische Modell gesteuert wird.

**6.** Verfahren nach Anspruch 1, wobei das Bestimmen der kardiovaskulären systemischen Impedanz und der kardiovaskulären pulmonalen Impedanz, die für den Patienten personalisiert sind, ein Bestimmen mit Induktivität von arteriellen Nebenhöhlen, Aortenarterien und/oder pulmonalen Arterien und/oder ein Bestimmen mit Widerständen des arteriellen Baumes umfasst.

**7.** Verfahren nach Anspruch 1, wobei das Bestimmen der zeitvariablen Flussrate für das Herz und der Druckänderung für das Herz ein Bestimmen eines Modells der Herzklappendynamik umfasst.

**8.** Verfahren nach Anspruch 1, wobei das kardiovaskuläre Systemmodell ein kardiovaskuläres Systemmodell mit einem geschlossenen Kreislauf ist.

**9.** Verfahren nach Anspruch 8, ferner umfassend ein Ändern von Parametern des kardiovaskulären Systemmodells mit einem geschlossenen Kreislauf basierend auf einem der genannten einen oder mehreren Regulierungssystemmodellen.

**10.** Verfahren nach Anspruch 9, wobei das Ändern ein Ändern mit dem einen von dem einen oder den mehreren Regulierungssystemmodellen umfasst, die ein Baroreflex-Systemmodell umfassen, das mit dem kardiovaskulären Systemmodell mit einem geschlossenen Kreislauf gekoppelt ist.

**11.** Verfahren nach Anspruch 1, wobei das Berechnen der Metrik ein Berechnen einer Druck-Volumen-Schleife einer Herzkammer, einer Schlaganfall-Belastung, einer arteriell-ventrikulären Kopplung, eines isochronen Volumenfußes und/oder einer myokardialen Belastung umfasst.

**12.** Verfahren nach Anspruch 1, ferner umfassend:

Durchführen einer Sensitivitätsanalyse von Parametern des mehrskaligen Ganzkörper-Kreislaufmodells für den Patienten;
Personalisieren einer Teilmenge der Parameter, die basierend auf der Sensitivitätsanalyse ausgewählt werden; und
Ausführen eines Vorwärtsmodells des mehrskaligen Ganzkörper-Kreislaufmodells mit den personalisierten Parametern der Teilmenge.

**13.** Verfahren nach Anspruch 12, wobei das Personalisieren ein Lösen für die Parameter basierend auf einer Differenz zwischen gemessenen und modellierten Werten umfasst.

## Revendications

**1.** Procédé de calcul personnalisé de la circulation du corps entier, le procédé comprenant :

la saisie des données spatiales cardiovasculaires d'un patient avec un scanner médical ;
la saisie des données d'électrophysiologie cardiaque du patient avec un capteur d'électrophysiologie cardiaque ;
la saisie des données de pression du patient avec un capteur de pression ;
la mesure d'un paramètre hémodynamique cardiaque à partir des données spatiales cardiovasculaires ;
la détermination d'un débit variant dans le temps pour le cœur, d'une variation de pression pour le cœur, d'une impédance systémique cardiovasculaire et d'une impédance pulmonaire cardiovasculaire personnalisés pour le patient à partir des données spatiales cardiovasculaires, des données ECG et des données de pression ;
le calcul d'une métrique avec un modèle de circulation du corps entier à plusieurs échelles en fonction du débit variant dans le temps pour le cœur, de la variation de pression du cœur, de l'impédance systémique cardiovasculaire et de l'impédance pulmonaire cardiovasculaire personnalisés du patient ;
dans lequel le modèle de circulation du corps entier à plusieurs échelles comprend un modèle de système cardiovasculaire couplé à un ou plusieurs modèles représentant les systèmes de régulation cardiovasculaires, et un échange d'informations bidirectionnel entre les systèmes conduit à une adaptation continue de l'activité et du fonctionnement cardiovasculaires, dans lequel les données de variation du débit ou du volume et de la pression provenant du modèle de système cardiovasculaire sont fournies à un ou plusieurs modèles de système de régulation et le ou les modèles de système de régulation renvoient des valeurs adaptées ou modifiées pour une utilisation dans le modèle de système cardiovasculaire, et
indiquant la métrique sur un affichage pour le patient.

2. Procédé selon la revendication 1, dans lequel la saisie des données spatiales cardiovasculaires comprend la saisie des données ultrasonores du cœur à l'aide du scanner médical comprenant un scanner à ultrasons.

3. Procédé selon la revendication 1, comprenant en outre la segmentation des données spatiales cardiovasculaires pour un cœur du patient dans au moins deux phases d'un cycle cardiaque.

4. Procédé selon la revendication 1, dans lequel le modèle de circulation du corps entier à plusieurs échelles comprend une combinaison d'un modèle localisé et d'un modèle tridimensionnel d'au moins une partie du cœur, et dans lequel la détermination comprend la détermination à l'aide d'un modèle anatomique, d'un modèle hémodynamique, d'un modèle électrophysiologique et d'un modèle biomécanique personnalisé pour le patient.

5. Procédé selon la revendication 4, dans lequel la détermination avec le modèle biomécanique comprend la détermination, avec les composants actifs et passifs du modèle biomécanique, du composant actif contrôlé par le modèle électrophysiologique.

6. Procédé selon la revendication 1, dans lequel la détermination de l'impédance systémique cardiovasculaire et de l'impédance pulmonaire cardiovasculaire personnalisées pour le patient comprend la détermination avec inductance des sinus artériels, des artères aortiques et/ou des artères pulmonaires et/ou la détermination avec des résistances de l'arbre artériel.

7. Procédé selon la revendication 1, dans lequel la détermination du débit variant dans le temps pour le cœur et de la variation de pression pour le cœur comprend la détermination d'un modèle de la dynamique de la valvule cardiaque.

8. Procédé selon la revendication 1, dans lequel ledit modèle de système cardiovasculaire est un modèle de système cardiovasculaire en boucle fermée.

9. Procédé selon la revendication 8, comprenant en outre la modification de paramètres du modèle de système cardiovasculaire en boucle fermée sur la base de l'un desdits un ou plusieurs modèles de système de régulation.

10. Procédé selon la revendication 9, dans lequel la modification comprend la modification avec l'un desdits un ou plusieurs modèles de système de régulation comprenant un modèle de système baroréflexe couplé au modèle de système cardiovasculaire à boucle fermée.

11. Procédé selon la revendication 1, dans lequel le calcul de la métrique comprend le calcul d'une boucle pression-volume d'un ventricule, d'une charge de travail systolique, d'un couplage ventriculo-artériel, d'un pied volumique d'isochrones et/ou d'une déformation du myocarde.

12. Procédé selon la revendication 1, comprenant en outre :

la réalisation d'une analyse de sensibilité des paramètres du modèle de circulation du corps entier à plusieurs échelles pour le patient ;
la personnalisation d'un sous-ensemble des paramètres sélectionnés sur la base de l'analyse de sensibilité ; et
l'exécution d'un modèle avancé du modèle de circulation du corps entier à plusieurs échelles avec les paramètres personnalisés du sous-ensemble.

**13.** Procédé selon la revendication 12, dans lequel la personnalisation comprend la résolution des paramètres sur la base d'une différence entre les valeurs mesurées et modélisées.

FIG 1

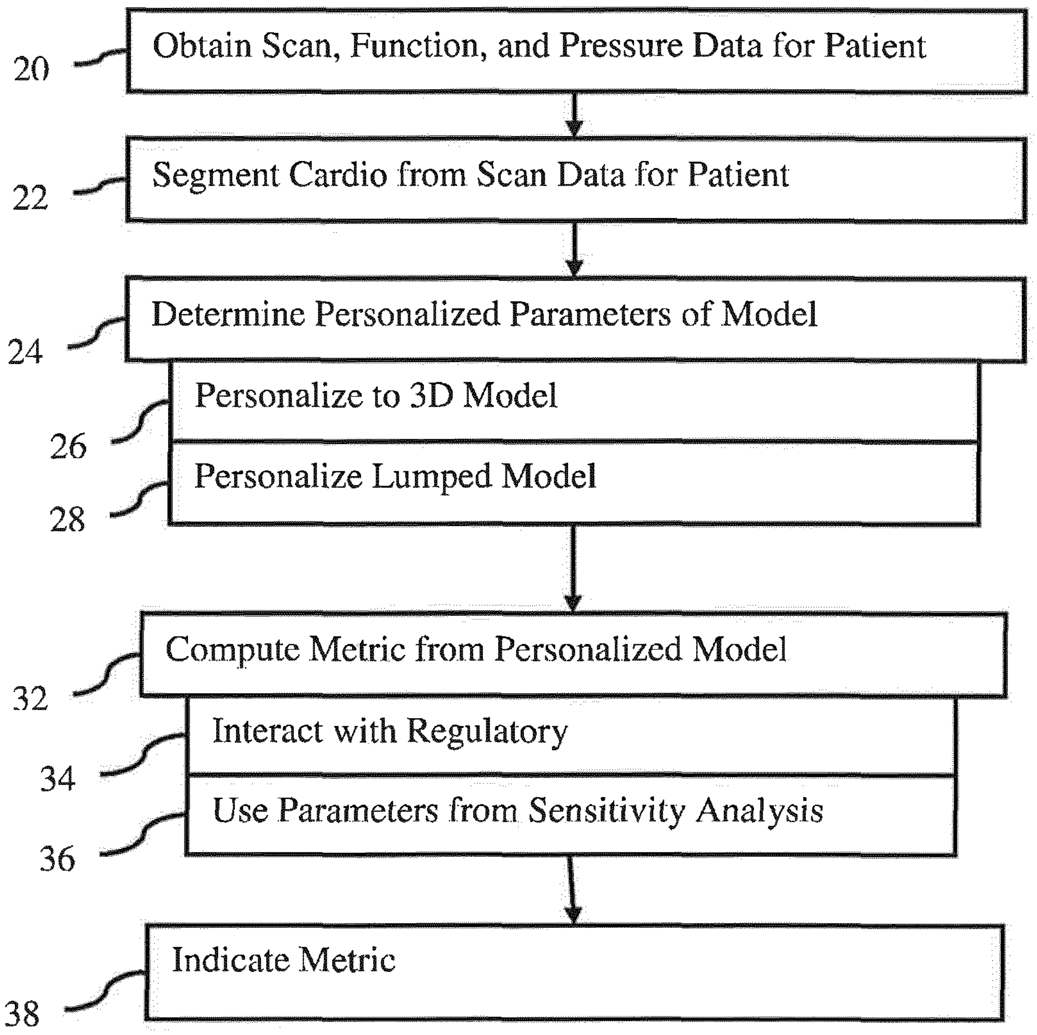
20
Obtain Scan, Function, and Pressure Data for Patient
22
Segment Cardio from Scan Data for Patient
24
Determine Personalized Parameters of Model
26
Personalize to 3D Model
28
Personalize Lumped Model
32
Compute Metric from Personalized Model
34
Interact with Regulatory
36
Use Parameters from Sensitivity Analysis
38
Indicate Metric

FIG 2

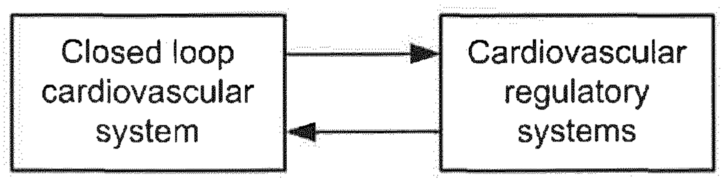
Closed loop cardiovascular system
Cardiovascular regulatory systems

FIG 3

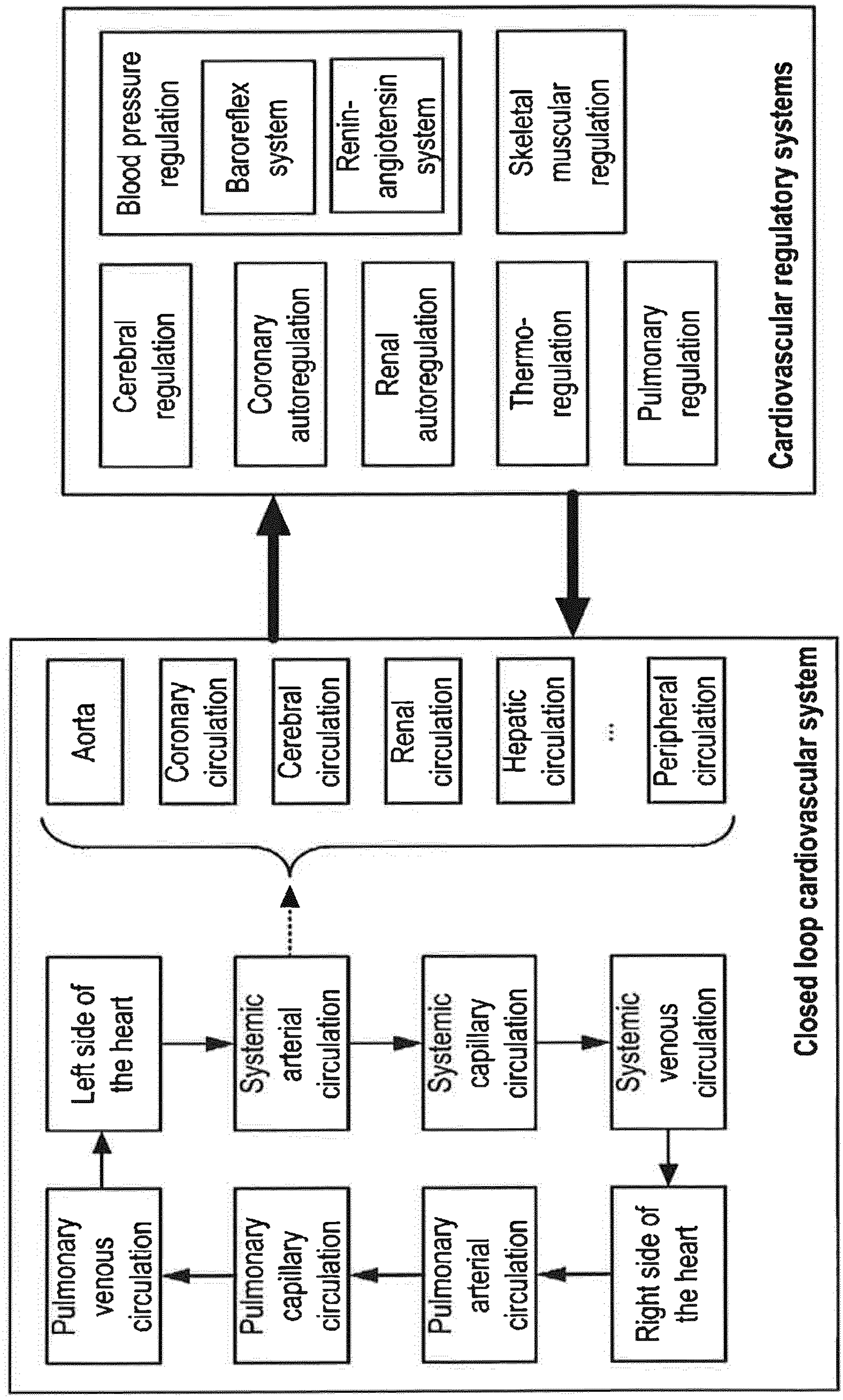
Blood pressure regulation
Baroreflex system
Renin-angiotensin system
Skeletal muscular regulation
Cerebral regulation
Coronary autoregulation
Renal autoregulation
Thermo-regulation
Pulmonary regulation
Cardiovascular regulatory systems
Aorta
Coronary circulation
Cerebral circulation
Renal circulation
Hepatic circulation
...
Peripheral circulation
Left side of the heart
Systemic arterial circulation
Systemic capillary circulation
Systemic venous circulation
Pulmonary venous circulation
Pulmonary capillary circulation
Pulmonary arterial circulation
Right side of the heart
Closed loop cardiovascular system

FIG 4

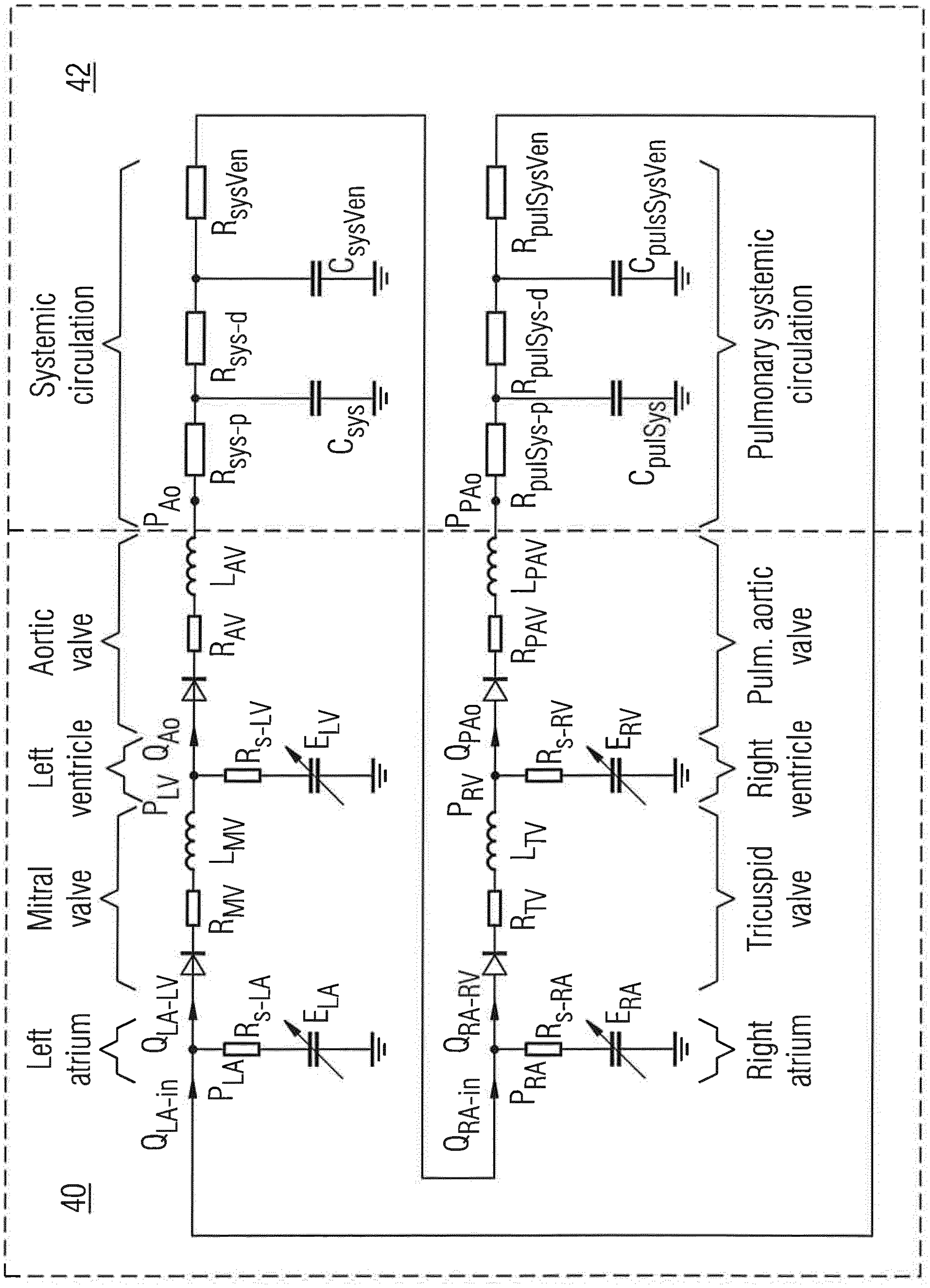
40
42
Left atrium
Mitral valve
Left ventricle
Aortic valve
Systemic circulation
Right atrium
Tricuspid valve
Right ventricle
Pulm. aortic valve
Pulmonary systemic circulation
Q_LA-in
P_LA
R_S-LA
E_LA
Q_LA-LV
R_MV
L_MV
P_LV
Q_Ao
R_S-LV
E_LV
R_AV
L_AV
P_Ao
R_sys-p
C_sys
R_sys-d
C_sysVen
R_sysVen
Q_RA-in
P_RA
R_S-RA
E_RA
Q_RA-RV
R_TV
L_TV
P_RV
Q_PAo
R_S-RV
E_RV
R_PAV
L_PAV
P_PAo
R_pulSys-p
C_pulSys
R_pulSys-d
C_pulsSysVen
R_pulSysVen

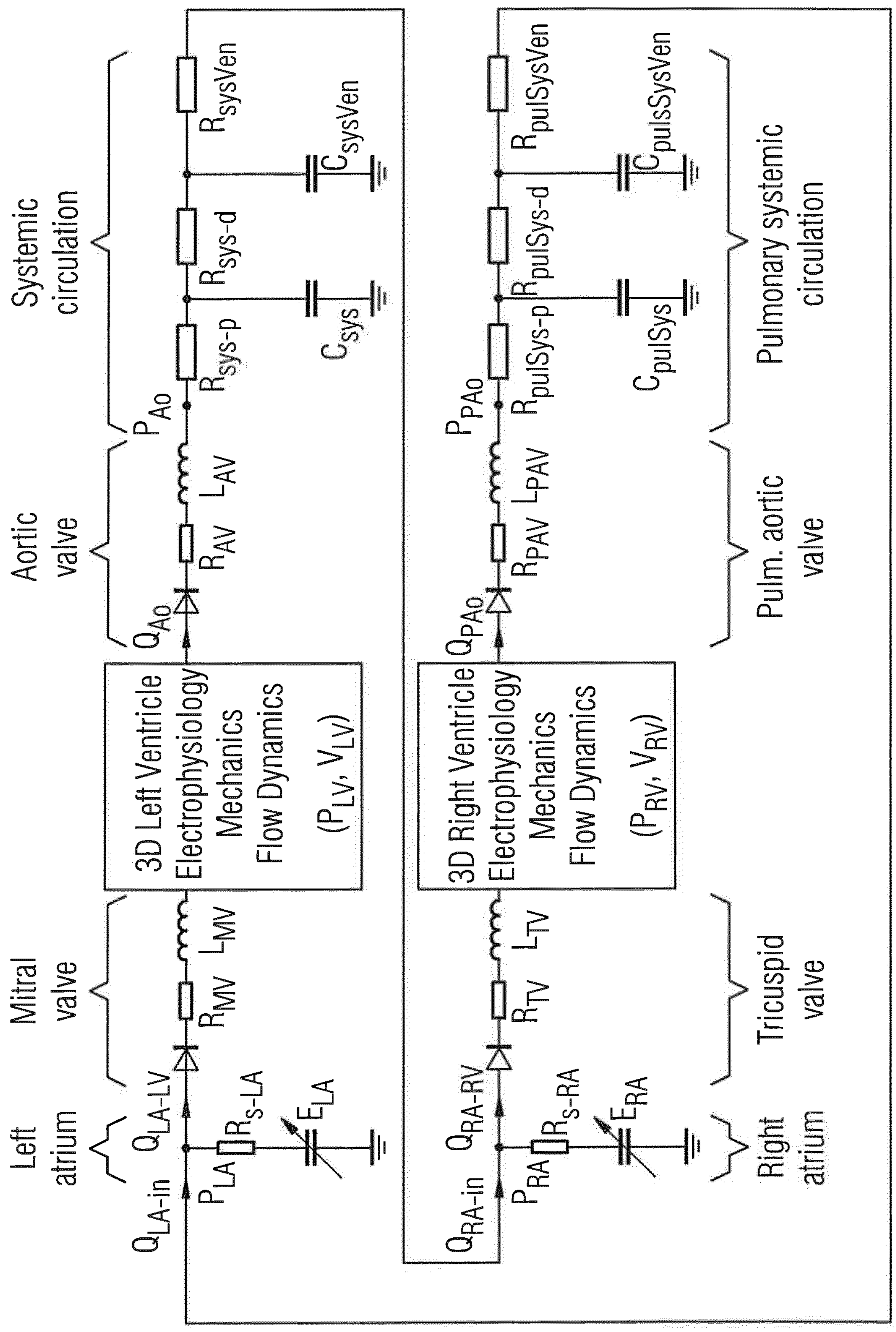
Left atrium
Mitral valve
Aortic valve
Systemic circulation
$Q_{LA-in}$
$P_{LA}$
$Q_{LA-LV}$
$R_{S-LA}$
$E_{LA}$
$R_{MV}$
$L_{MV}$
3D Left Ventricle
Electrophysiology
Mechanics
Flow Dynamics
$(P_{LV}, V_{LV})$
$Q_{Ao}$
$R_{AV}$
$L_{AV}$
$P_{Ao}$
$R_{sys-p}$
$C_{sys}$
$R_{sys-d}$
$C_{sysVen}$
$R_{sysVen}$
$Q_{RA-in}$
$P_{RA}$
$Q_{RA-RV}$
$R_{S-RA}$
$E_{RA}$
$R_{TV}$
$L_{TV}$
3D Right Ventricle
Electrophysiology
Mechanics
Flow Dynamics
$(P_{RV}, V_{RV})$
$Q_{PAo}$
$R_{PAV}$
$L_{PAV}$
$P_{PAo}$
$R_{pulSys-p}$
$C_{pulSys}$
$R_{pulSys-d}$
$C_{pulsSysVen}$
$R_{pulSysVen}$
Right atrium
Tricuspid valve
Pulm. aortic valve
Pulmonary systemic circulation

FIG 5

FIG 6

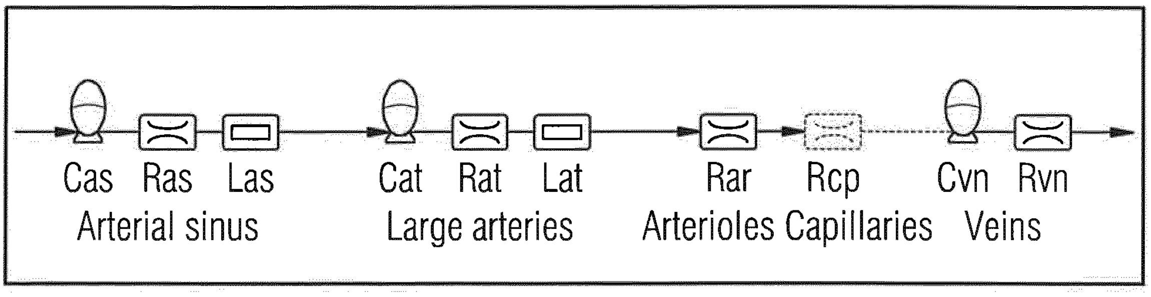
Cas Ras Las
Arterial sinus
Cat Rat Lat
Large arteries
Rar
Arterioles
Rcp
Capillaries
Cvn Rvn
Veins

FIG 7

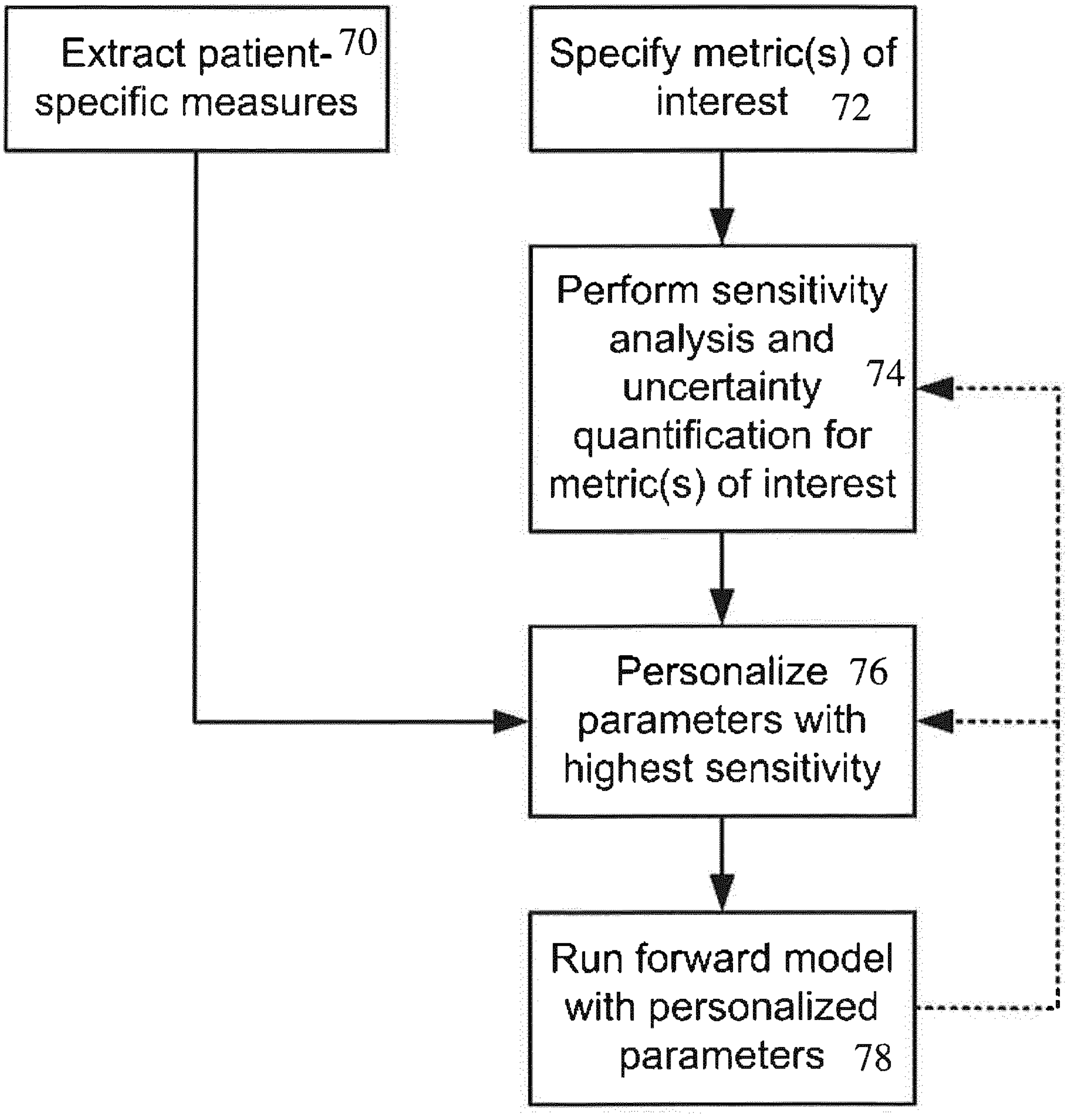
Extract patient-specific measures 70
Specify metric(s) of interest 72
Perform sensitivity analysis and uncertainty quantification for metric(s) of interest 74
Personalize parameters with highest sensitivity 76
Run forward model with personalized parameters 78

FIG 8

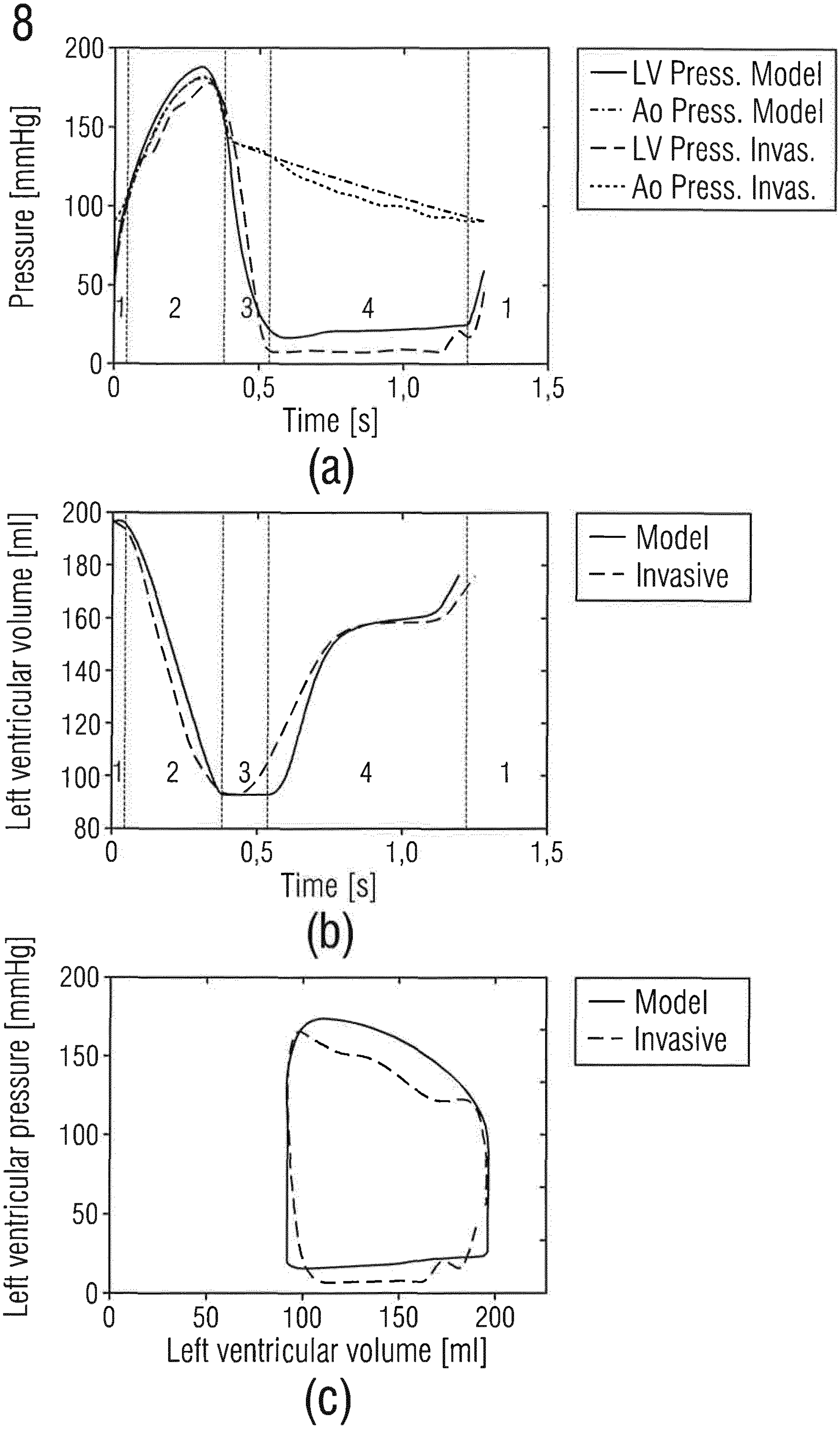
Pressure [mmHg]
200
150
100
50
0
0
0,5
1,0
1,5
Time [s]
1
2
3
4
1
LV Press. Model
Ao Press. Model
LV Press. Invas.
Ao Press. Invas.
(a)
Left ventricular volume [ml]
200
180
160
140
120
100
80
0
0,5
1,0
1,5
Time [s]
1
2
3
4
1
Model
Invasive
(b)
Left ventricular pressure [mmHg]
200
150
100
50
0
0
50
100
150
200
Left ventricular volume [ml]
Model
Invasive
(c)

FIG 9

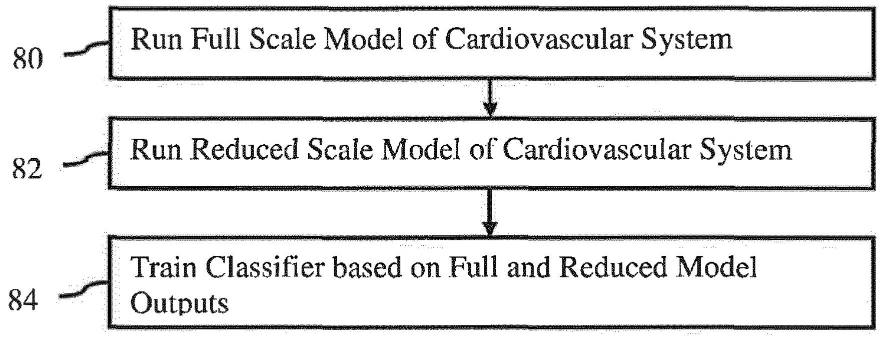
80
Run Full Scale Model of Cardiovascular System
82
Run Reduced Scale Model of Cardiovascular System
84
Train Classifier based on Full and Reduced Model Outputs

FIG 10

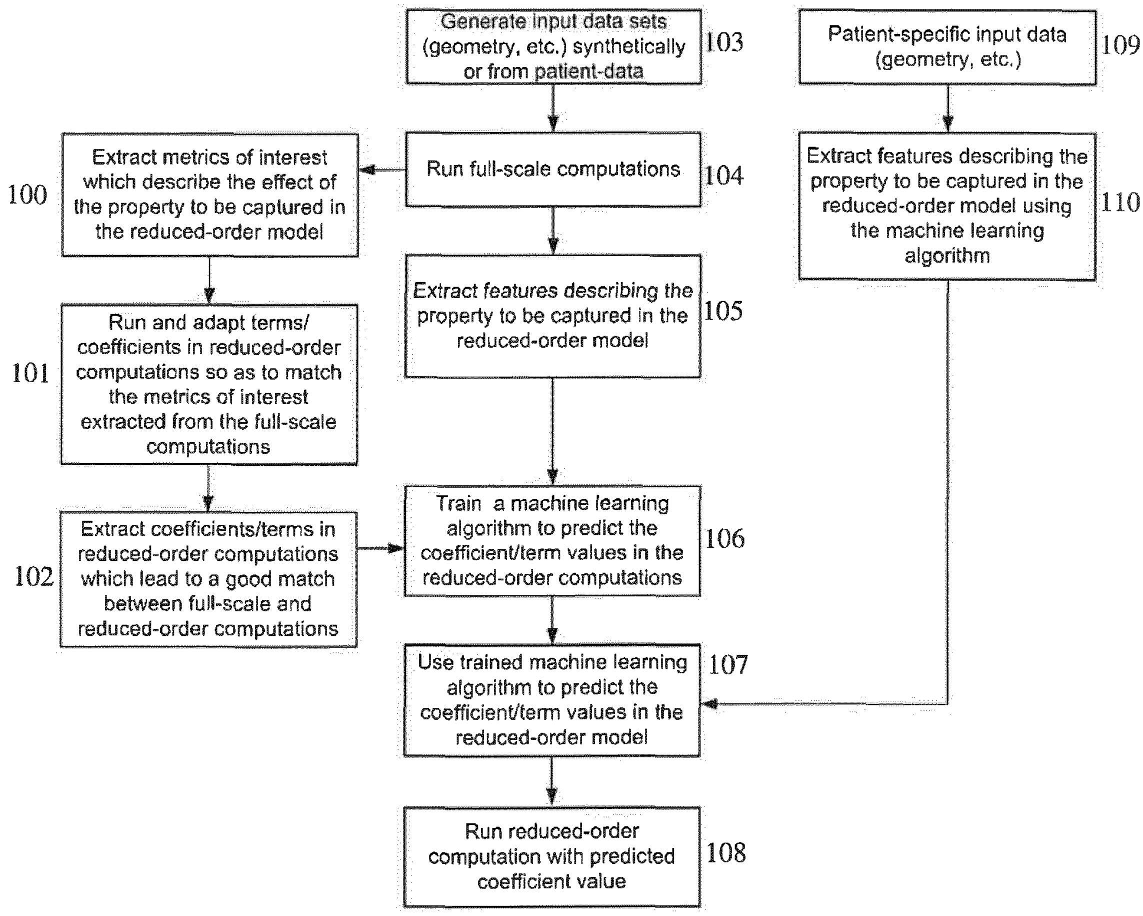
Generate input data sets (geometry, etc.) synthetically or from patient-data
103
Patient-specific input data (geometry, etc.)
109
Extract metrics of interest which describe the effect of the property to be captured in the reduced-order model
100
Run full-scale computations
104
Extract features describing the property to be captured in the reduced-order model using the machine learning algorithm
110
Run and adapt terms/ coefficients in reduced-order computations so as to match the metrics of interest extracted from the full-scale computations
101
Extract features describing the property to be captured in the reduced-order model
105
Extract coefficients/terms in reduced-order computations which lead to a good match between full-scale and reduced-order computations
102
Train a machine learning algorithm to predict the coefficient/term values in the reduced-order computations
106
Use trained machine learning algorithm to predict the coefficient/term values in the reduced-order model
107
Run reduced-order computation with predicted coefficient value
108

FIG 11

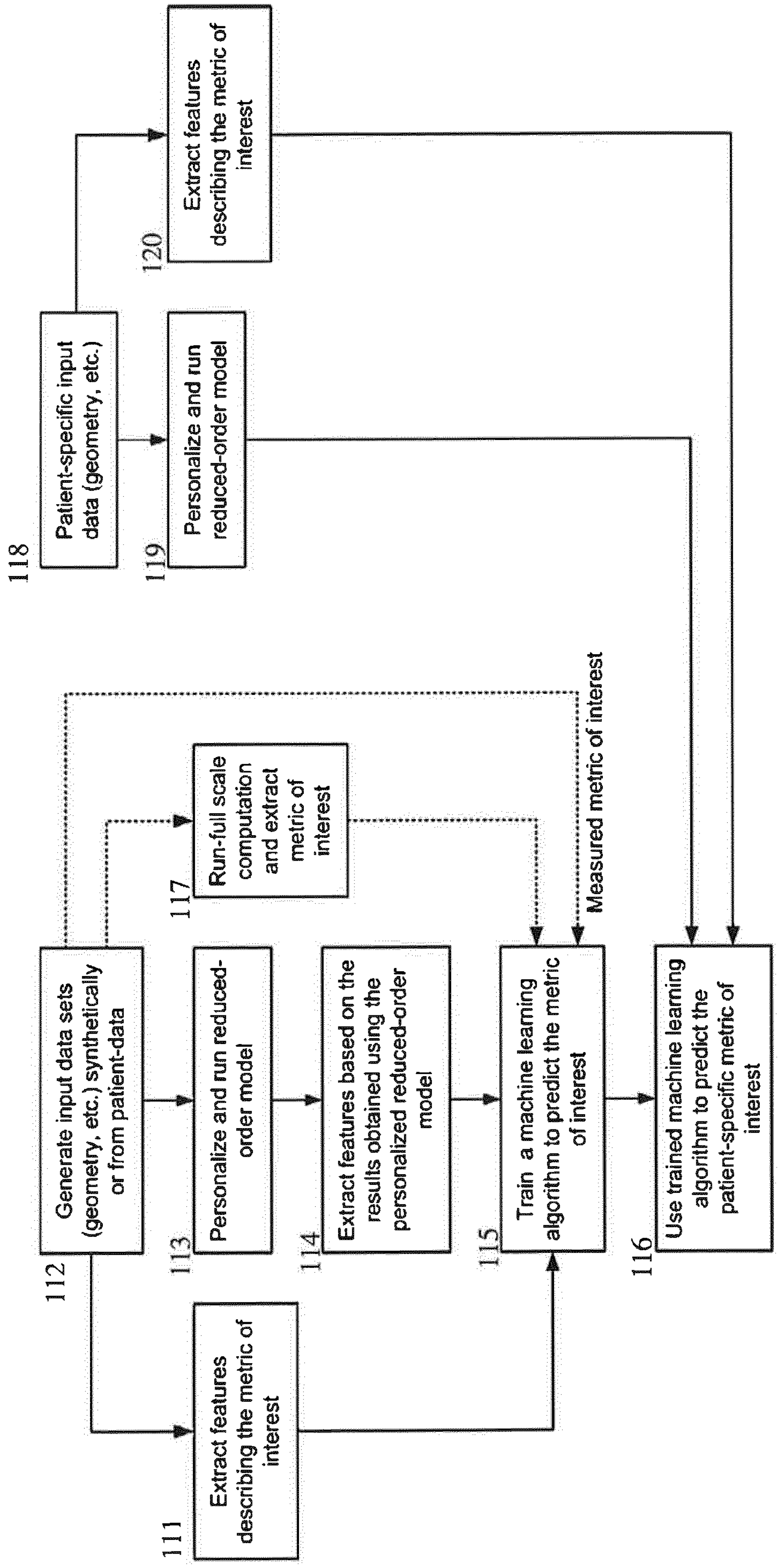
111 Extract features describing the metric of interest
112 Generate input data sets (geometry, etc.) synthetically or from patient-data
113 Personalize and run reduced-order model
114 Extract features based on the results obtained using the personalized reduced-order model
115 Train a machine learning algorithm to predict the metric of interest
116 Use trained machine learning algorithm to predict the patient-specific metric of interest
117 Run-full scale computation and extract metric of interest
Measured metric of interest
118 Patient-specific input data (geometry, etc.)
119 Personalize and run reduced-order model
120 Extract features describing the metric of interest

FIG 12

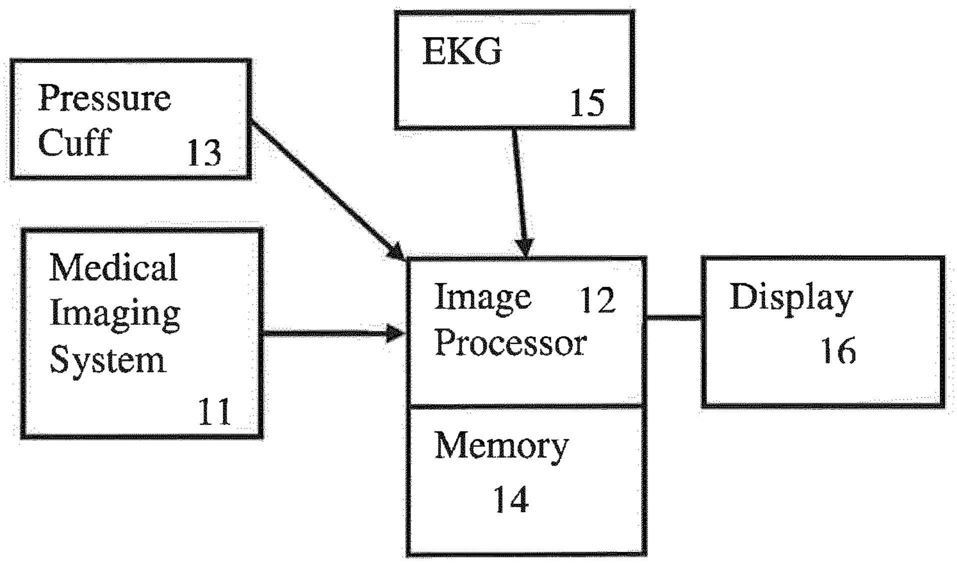
EKG
15
Pressure Cuff 13
Medical Imaging System 11
Image Processor 12
Display 16
Memory 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62100271


### Non-patent literature cited in the description


- **RADOMIR CHABINIOK.** *Personalized Biomechanical Heart Modeling for Clinical Applications,* 24 January 2011